# EUROPEAN PATENT SPECIFICATION

(11) **EP 1 576 143 B1**
(45) Date of publication and mention of the grant of the patent: **24.11.2010**
(21) Application number: 03808386.1
(22) Date of filing: 19.05.2003
(51) Int. Cl.: A61K 39/12

(54) **VIRUS-LIKE PARTICLES, METHODS OF PREPARATION, AND IMMONOGENIC COMPOSITIONS**
VIRUSÄHNLICHE PARTIKEL, VERFAHREN ZUR HERSTELLUNG UND IMMUNOGENE ZUSAMMENSETZUNGEN
PARTICULES PSEUDOVIRALES, PROCEDE DE FABRICATION ET COMPOSITIONS IMMUNOGENIQUES

(30) Priority: 17.05.2002 US 381557 P; 11.03.2003 US 454115 P; 11.03.2003 US 454139 P; 14.03.2003 US 454584 P; 06.05.2003 US 468318 P; 16.05.2003 US 471246 P
(43) Date of publication of application: 21.09.2005
(73) Proprietor: Emory University, Atlanta, Georgia 30322 (US)
(72) Inventor: COMPANS, Richard, W., Atlanta, GA 30329 (US); YANG, Chinglai, Decatur, CA 30030 (US); YAO, Qizhi, Houston, TX 77584 (US); KANG, Sang-Moo, Norcross, GA 30092 (US)
(74) Representative: Weiss, Wolfgang
(86) International application number: PCT/US2003/015930
(87) International publication number: WO 2004/042001

(56) References cited:
- US-A- 5 618 536
- US-A- 5 714 374
- US-A1- 2001 016 199
- US-A1- 2005 186 621
- US-B1- 6 171 591
- YAO QIZHI ET AL: "Production and characterization of simian-human immunodeficiency virus-like particles" AIDS RESEARCH AND HUMAN RETROVIRUSES, vol. 16, no. 3, 10 February 2000 (2000-02-10), pages 227-236, XP002437946 ISSN: 0889-2229
- LATHAM T ET AL: "Formation of wild-type and chimeric influenza virus-like particles following simultaneous expression of only four structural proteins" JOURNAL OF VIROLOGY, THE AMERICAN SOCIETY FOR MICROBIOLOGY, US, vol. 75, no. 13, July 2001 (2001-07), pages 6154-6165, XP002323947 ISSN: 0022-538X
- MCGETTIGAN JAMES P ET AL: "Rabies virus-based vectors expressing human immunodeficiency virus type 1 (HIV-1) envelope protein induce a strong, cross-reactive cytotoxic T-lymphocyte response against envelope proteins from different HIV-1 isolates" JOURNAL OF VIROLOGY, vol. 75, no. 9, May 2001 (2001-05), pages 4430-4434, XP002437947 ISSN: 0022-538X
- SCHNELL MATTHIAS J ET AL: "Recombinant rabies virus as potential live-viral vaccines for HIV-1" PROCEEDINGS OF THE NATIONAL ACADEMY OF SCIENCES OF THE UNITED STATES OF AMERICA, vol. 97, no. 7, 28 March 2000 (2000-03-28), pages 3544-3549, XP002313400 ISSN: 0027-8424
- YAMSHCHIKOV G V ET AL: "ASSEMBLY OF SIV VIRUS-LIKE PARTICLES CONTAINING ENVELOPE PROTEINS USING A BACULOVIRUS EXPRESSION SYSTEM" VIROLOGY, ACADEMIC PRESS,ORLANDO, US, vol. 214, no. 1, 1 December 1995 (1995-12-01), pages 50-58, XP000608778 ISSN: 0042-6822
- GUO L ET AL: "Enhancement of mucosal immune responses by chimeric influenza HA/SHIV virus-like particles" VIROLOGY, ACADEMIC PRESS,ORLANDO, US, vol. 313, no. 2, 1 September 2003 (2003-09-01), pages 502-513, XP004452429 ISSN: 0042-6822
- MCGETTIGAN JAMES P ET AL: "Expression and immunogenicity of human immunodeficiency virus type 1 Gag expressed by a replication-competent rhabdovirus-based vaccine vector" JOURNAL OF VIROLOGY, vol. 75, no. 18, September 2001 (2001-09), pages 8724-8732, XP002437949 ISSN: 0022-538X
- GARNIER LAURENCE ET AL: "Incorporation of pseudorabies virus gD into human immunodeficiency virus type 1 Gag particles produced in baculovirus-infected cells" JOURNAL OF VIROLOGY, vol. 69, no. 7, 1995, pages 4060-4068, XP002437950 ISSN: 0022-538X
- SALMONS ET AL: 'Construction of retroviral vectors for targeted delivery and expression of therapeutic genes.' LEUKEMIA. vol. 9, no. 1, 1995, pages S53 - S60, XP001037672
- PUSHLO ET AL: 'Individual and bivalent vaccines based on alphavirus replicons protect guinea pigs against infection with Lassa and Ebola viruses.' JOURNAL OF VIROLOGY. vol. 75, no. 23, December 2001, pages 11677 - 11685, XP002982005
- MCGUIGAN ET AL: 'Recombinant-expressed virus-like particle pseudotypes as an approach to vaccine development.' VACCINE. vol. 11, no. 6, 1993, pages 675 - 678, XP002993272
- KANG ET AL: 'Mucosal immunization with virus-like particles of simian immunodeficiency virus conjugated with cholera toxin subunit B.' JOURNAL OF VIROLOGY. vol. 77, no. 18, September 2003, pages 9823 - 9830, XP002993273
- SCHMITT ET AL: 'Requirements for budding of paramyxovirus simian virus 5 virus-like particles.' JOURNAL OF VIROLOGY. vol. 76, no. 8, April 2002, pages 3952 - 3964, XP002993274
- NODA ET AL: 'Ebola virus VP40 drives the formation of virus-like filamentous particles along with GP.' JOURNAL OF VIROLOGY. vol. 76, no. 10, May 2002, pages 4855 - 4865, XP002976165
- RABU ET AL: 'Chimeric Newcastle disease virus nucleocapsid with parts of viral hemagglutinin-neuraminidase and fusion proteins.' ACTA VIROLOGY. vol. 46, no. 4, 2002, pages 211 - 217, XP008067212
- MCINERNEY ET AL: 'Analysis of the ability of five adjuvants to enhance immune responses to a chimeric plant virus displaying an HIV-1 peptide.' VACCINE. vol. 17, no. 11-12, 1999, pages 1359 - 1368, XP004158263
- GODEKE ET AL: 'Assembly of spikes into coronavirus particles is mediated by the carboxy-terminal domain of the spike protein.' JOURNAL OF VIROLOGY. vol. 74, no. 3, 2000, pages 1566 - 1571, XP002960963

## Description

### STATEMENT REGARDING FEDERALLY SPONSORED

### RESEARCH OR DEVELOPMENT

The U.S. government may have a paid-up license in part of this invention and the right in limited circumstances to require the patent owner to license others on reasonable terms as provided for by the terms of 1R21 AI53514, 1R21 AI44409, and 5R21 AI049116, which all were awarded by the National Institute of Health.

### TECHNICAL FIELD

The present invention is generally related to virus-like particles, methods of preparing virus-like particles, immunogenic compositions that include virus-like particles, and methods of eliciting an immune response using immunogenic compositions that include virus-like particles.

### BACKGROUND

Virus-like particles (VLPs) closely resemble mature virions, but they do not contain viral genomic material (*i*.*e*., viral genomic RNA). Therefore, VLPs are nonreplicative in nature, which make them safe for administration in the form of an immunogenic composition (*e.g*., vaccine). In addition, VLPs can express envelope glycoproteins on the surface of the VLP, which is the most physiological configuration. Moreover, since VLPs resemble intact virions and are multivalent particulate structures, VLPs may be more effective in inducing neutralizing antibodies to the envelope glycoprotein than soluble envelope antigens. Further, VLPs can be administered repeatedly to vaccinated hosts, unlike many recombinant vaccine approaches. An example of a VLP vaccine is the baculovirus-derived recombinant human papillomavirus type (HPV-16) L1 VLP, which was manufactured by Novavax, Inc.

Therefore, VLPs can be used to overcome previous attempts to create vaccines for various viruses such as human immunodeficiency virus (HIV), Ebola virus, severe acute respiratory syndrome (SARS), coronavirus, and Rift Valley Fever virus (RVFV).

### SUMMARY

Briefly described, embodiments of the present invention include novel types of virus-like particles, methods of preparing virus-like particles, immunogenic compositions that include virus-like particles, and methods of eliciting an immune response using immunogenic compositions that include virus-like particles. One exemplary embodiment of a novel type of virus-like particle includes virus-like particle (VLP) that include a viral core protein that can self-assemble into the VLP core and at least one viral surface envelope glycoprotein expressed on the surface of the VLP. The viral protein and the viral surface envelope glycoprotein are from different viruses. Another exemplary embodiment a VLP includes a VLP having a viral core protein that can self assemble into a VLP core; at least one viral surface envelope glycoprotein expressed on the surface of the VLP; and at least one adjuvant molecule expressed on the surface of the VLP.

Another representative embodiment of the present invention includes an immunogenic composition. The immunogenic composition includes a pharmacologically acceptable carrier and at least one of the VLPs described above. Further, another representative embodiment includes a method of generating an immunological response in a host by administering an effective amount of one or more of the immunogenic compositions described above to the host. Furthermore, another representative embodiment includes a method of treating a condition by administering to a host in need of treatment an effective amount of one or more of the immunogenic compositions described above.

Still another representative embodiment of the present invention includes methods of determining exposure of a host to a virus. An exemplary method, among others, includes the steps of: contacting a biological fluid of the host with one or more of the VLPs discussed above, wherein the VLP is of the same virus type to which exposure is being determined, under conditions which are permissive for binding of antibodies in the biological fluid with the VLP; and detecting binding of antibodies within the biological fluid with the VLP, whereby exposure of the host to the virus is determined by the detection of antibodies bound to the VLP.

Still another representative embodiment of the present invention includes methods of making VLPs. An exemplary method, among others, includes the steps of: providing a viral core protein expression vector; providing a viral surface envelope glycoprotein expression vector; providing a adjuvant molecule expression vector; and introducing into a cell the viral core protein expression vector, the viral surface envelope glycoprotein expression vector, and the adjuvant molecule expression vector and allowing for expression of the viral surface envelope glycoprotein and the adjuvant molecule, whereby the VLP is formed by the cells.

Other systems, methods, features, and advantages of the present invention will be or will become apparent to one with skill in the art upon examination of the following drawings and detailed description. It is intended that all such additional systems, methods, features, and advantages be included within this description, be within the scope of the present invention, and be protected by the accompanying claims.

### BRIEF DESCRIPTION OF THE DRAWINGS

Many aspects of the invention can be better understood with reference to the following drawings. The components in the drawings are not necessarily to scale, emphasis instead being placed upon clearly illustrating the principles of the present invention. Moreover, in the drawings, like reference numerals designate corresponding parts throughout the several views.
FIG. 1 illustrates a representative virus-like particle (VLP).
FIG. 2 illustrates some representative structural changes that can be made to a representative viral surface envelope glycoprotein.
FIG. 3 illustrates some representative structural changes that can be made to another representative viral surface envelope glycoprotein.
FIGS. 4A and 4B illustrate western blots of representative VLPs incorporating HIV envelope glycoproteins into SHIV VLPs (FIG. 4A) and influenza HA adjuvant molecules into SHIV VLPs (FIG. 4B).
FIGS. 5A through 5B illustrate graphs measuring various characteristics of a number of VLPs that were intranasally introduced into mice. In FIGS. 5A through 5D, "V" represents SHIV VLPs, "HA/V" represents HA/SHIV VLPs, "V+CT" represents SHIV VLPs (10 µg) + CT (10 µg), and "N" represents a negative control (PBS). In addition, the number in parentheses indicates the "µg's" of VLPs used for the immunization of the mice.

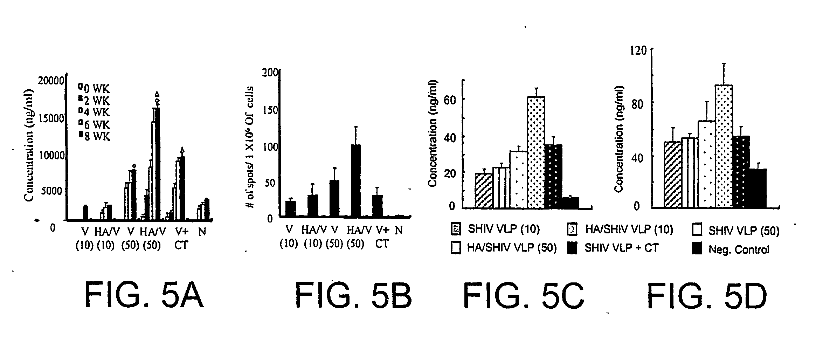

FIG. 5A illustrates a graph measuring serum IgG levels specific to HIV envelope glycoproteins. FIG. 5B illustrates a graph measuring splenocytes producing IFN-γ determined by ELISPOT assay. FIG. 5C illustrates a graph measuring HIV envelope glycoprotein-specific IgA in vaginal wash. FIG. 5D illustrates a graph measuring HIV envelope glycoprotein-specific IgA in fecal extracts.

### DETAILED DESCRIPTION

Embodiments of the present invention provide for virus-like particles, methods of using the virus-like particles, and methods of making virus-like particles that can be used in immunogenic compositions to treat conditions in a host, and the immunogenic compositions that include virus-like particles. The virus-like particles can be used to enhance immune responses (*e.g*., antibody production, cytotoxic T cell activity, and cytokine activity). In particular, virus-like particles can act as a prophylactic as a vaccine to prevent viral infections such as those caused by, for example, the human immunodeficiency virus (HIV), the Corona virus, the Ebola virus, the Rift Valley Fever virus, the Hantaan Virus, the Lassa fever virus, and the Flavivirus.

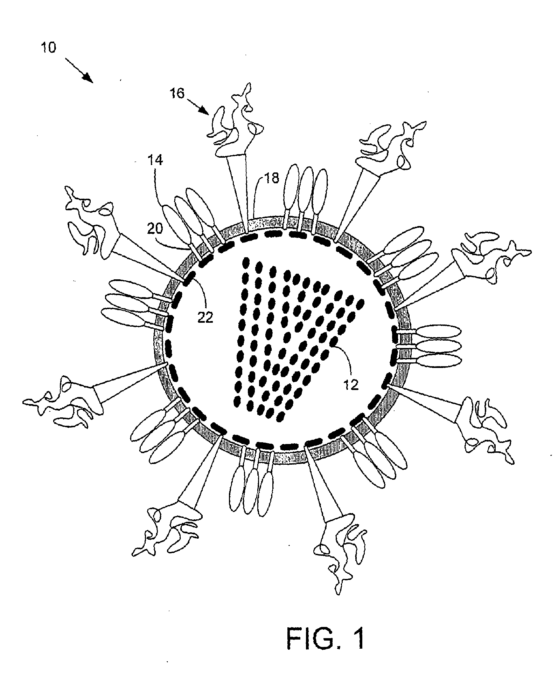

In general, the virus-like particle ("VLP") 10 includes at least a viral core protein 12 (hereinafter "viral protein") and at least one viral surface envelope glycoprotein 14 (*e.g*., type 1 or type 2 viral surface envelope glycoproteins), as shown in FIG. 1. In addition, the VLP can include at least one adjuvant molecule 16. Furthermore, the VLP can include a lipid membrane 18, viral glycoprotein transmembrane unit 20, and a matrix protein 22. In particular, chimeric VLPs are VLPs having at least one viral surface envelope glycoprotein incorporated into the VLP, wherein the viral core protein and the viral surface envelope glycoprotein are from different viruses. Furthermore, phenotypically mixed VLPs areVLPs having at least one adjuvant molecule incorporated into the VLP wherein the adjuvant molecule are from cells or viruses different than the viral protein.

Viral proteins 12 include proteins that are capable of self-assembling into the VLP (Freed, E.O., J. Virol., 76, 4679-87, (2002)). In particular, the viral proteins 12 can include, but are not limited to, a HIV Gag viral protein (*e.g*., HIV-1 NL43 Gag (GenBank serial number AAA44987)), simian immunodeficiency virus (SIV) Gag viral protein (*e*.*g*., SIVmac239 Gag (GenBank serial number CAA68379)), a murine leukemia virus (MuLV) Gag viral protein (*e.g*., MuLV Gag (GenBank serial number S70394)), a vesicular stomatis virus (VSV) M viral protein (*e.g*., VSV Matrix protein (GenBank serial number NP041714)), an Ebola VP40 viral protein (*e.g*., Ebola virus VP40 (GenBank serial number AAN37506)), a Rift Valley Fever viral N protein (*e.g*., RVFV N Protein (GenBank serial number NP049344)), a corona M and E virus core protein (GenBank serial number NP040838 for NP protein and NP 040835 for M protein)), a Bunya virus N Protein viral core protein (GenBank serial number AAA47114)), and combinations thereof. It may be necessary to include appropriate surface glycoproteins and/or viral RNA to form the VLP 10.

In general, the viral protein 12 sequence and the corresponding polynucleotide sequence can be found in GenBank and the access numbers can be obtained online at National Center for Biotechnology Information (NCBI). In addition, the sequences identified for the viral proteins 12 above are only illustrative examples of representative viral proteins 12. Furthermore, variants that are substantially homologous to the above referenced viral proteins 12 and viral proteins 12 having conservative substitutions of the above referenced viral proteins 12 can also be incorporated into VLPs 10 of the present invention to enhance the immunogenic characteristics of VLPs.

The viral surface envelope glycoprotein 14, or at least at portion of the viral surface envelope glycoprotein 14, is disposed (*e.g*., expressed) on the surface of the VLP. The viral surface envelope glycoprotein 14 is disposed on the surface of the VLP so that it can interact with target molecules or cells (*e.g*., the interaction between the HIV surface envelope glycoprotein and the B cell receptor to activate HIV envelope glycoprotein specific antibody producing B cells) to produce immunogenic responses (*e.g*., antibody production).

The viral surface envelope glycoproteins 14 can include, but are not limited to, a human immunodeficiency virus (HIV) envelope glycoprotein (*e.g*., HIVSF162 envelope glycoprotein (SEQ ID NO: 1, GenBank serial number M65024)), a simian immunodeficiency virus (SIV) envelope glycoprotein (*e.g*., SIVmac239 envelope glycoprotein (GenBank serial number M33262)), a simian-human immunodeficiency virus (SHIV) envelope glycoprotein (*e.g*., SHIV-89.6p envelope glycoprotein (GenBank serial number U89134)), a feline immunodeficiency virus (FIV) envelope glycoprotein (*e.g*., feline immunodeficiency virus envelope glycoprotein (GenBank serial number L00607)), a feline leukemia virus envelope glycoprotein (*e.g*., feline leukemia virus envelope glycoprotein (GenBank serial number M12500)), a bovine immunodeficiency virus envelope glycoprotein (*e.g*., bovine immunodeficiency virus envelope glycoprotein (GenBank serial number NC001413)), a bovine leukemia virus envelope glycoprotein (GenBank serial number AF399703), a equine infectious anemia virus envelope glycoprotein (*e.g*., equine infectious anemia virus envelope glycoprotein (GenBank serial number NC001450)), a human T-cell leukemia virus envelope glycoprotein (*e.g*., human T-cell leukemia virus envelope glycoprotein (GenBank serial number AF0033817)), a Rift Valley Fever virus (RVFV) glycoprotein, (*e.g*., RVFV envelope glycoprotein (SEQ ID NO: 2, GenBank serial number M11157)), a Bunya virus glycoprotein, Lassa fever virus glycoprotein (GenBank serial number AF333969)), Ebola virus glycoprotein (GenBank serial number NC002549)), corona virus glycoprotein (GenBank serial number SARS coronavirus spike protein AAP13567)), Arena virus glycoprotein (GenBank serial number AF333969)), Filovirus glycoprotein (GenBank serial number NC002549)), influenza virus glycoprotein (GenBank serial number V01085)), paramyxovirus glycoprotein (GenBank serial number NC002728 for Nipah virus F and G proteins)), rhabdovirus glycoprotein (GenBank serial number NP049548)), alphavirus glycoprotein (GenBank serial number AAA48370 for VEE), flavivirus glycoprotein (GenBank serial number NC001563 for West Nile virus)), and combinations thereof.

In general, the viral surface envelope glycoprotein 14 sequence and the corresponding polynucleotide sequence can be found in GenBank and the access numbers can be obtained online at NCBI. In addition, the sequences identified for the viral surface envelope glycoproteins 14 above are only illustrative examples of representative viral surface envelope glycoproteins 14. Further, variants that are substantially homologous to the above referenced viral surface envelope glycoproteins 14 and viral surface envelope glycoproteins 14 having conservative substitutions of the above referenced viral surface envelope glycoproteins 14 can also be incorporated into VLPs 10 of the present invention to enhance she immunogenic characteristics of VLPs. '

In one embodiment, the HIV envelope glycoprotein can be modified and/or truncated to improve the immunogenic properties of the VLP. In particular, the VLP can be conformationally changed by hydrostatic pressure-induced techniques.

In another embodiment, the HIV envelope glycoprotein can be modified to expose neutralizing epitopes in the HIV envelope glycoprotein by removing obstructing structural features such as, but not limited to, glycosylation sites, the V1 loop, the V2 loop, and the V3 loop. By eliminating these obstructing features, the immunogenic properties of the VLP can be enhanced.

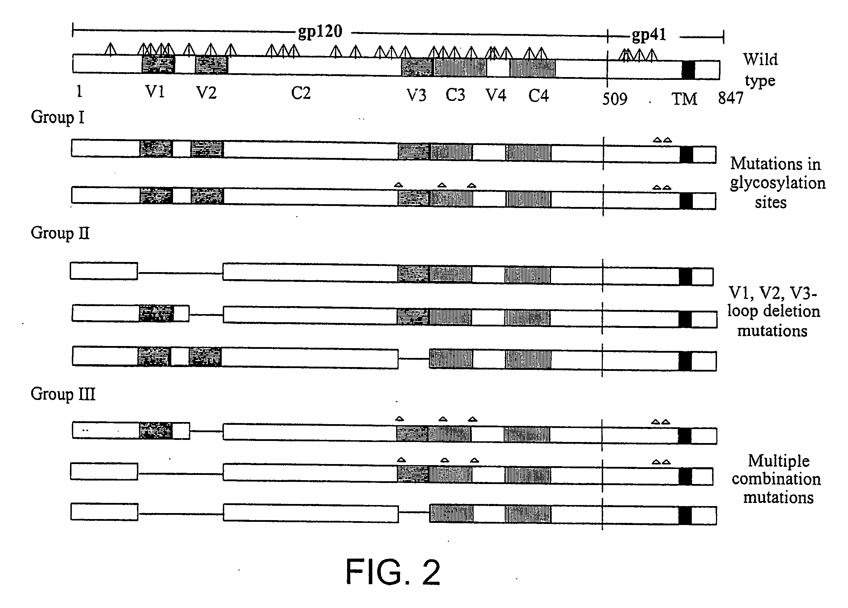

FIG. 2 illustrates some representative structural changes that can be made to the HIV 89.6 envelope glycoprotein (GenBank serial number AAA81043, SEQ ID NO: 3). The arrows in FIG. 2 indicate the N-glycosylation motifs in the HIV 89.9 viral surface envelope glycoprotein as well as the V1 (amino acids 128-164 of SEQ ID NO: 3) loop, V2 (amino acids 164-194 of SEQ ID NO: 3) loop, and V3 (amino acids 298-329 of SEQ ID NO: 3) loop domains. Deletions of the loops is shown by removing the corresponding sequence in the HIV 89.6 envelope glycoprotein shown in FIG. 2. When the loop is , deleted, a 3 amino acid linker (GAG) is inserted into the loops former position in the sequence. The glycosylation motifs mutated are denoted by triangles. Three glycosylation motif mutations of Asn to Gln in the V3/C3 (amino acids 298-402 of SEQ ID NO: 3) domains are performed on amino acids 301, 341, and 362. In the gp41 domain (amino acids 509-853 of SEQ ID NO: 3), the glycosylation motif mutations are performed on Asn in amino acid position 623 and 635. TM denotes the transmembrane domain (amino acids 681-7033 of SEQ ID NO: 3). In FIG. 2, Group I illustrates the glycosylation motif mutations in gp41, the V1 loop domains, the V2 loop domain, and the V3-C3 loop domain. Group II illustrates variable loop (V1, V2, and/or V3) deletion mutations, while Group III illustrates representative multiple combination mutations.

For example, mutations of glycosylation sites in gp41 can be performed to enhance the immunogenic properties of a VLP incorporating the HIV envelope glycoprotein. Although most of gp41 appears to be completely occluded in the HIV-1 envelope spike, recent studies indicate that regions of gp41 close to the transmembrane domain are accessible to neutralizing antibodies (Abs). Several mAbs (2F5, Z13, 4E10), which neutralize a broad range of primary HIV-1 isolates, are known to bind to the extracellular domain of gp41. However, attempts to elicit antibodies having these properties by immunization with linear peptide epitopes or with other carrier proteins containing peptide epitopes have not been successful (Coeffier et al., Vaccine, 19, 684-693, (2000); Eckhart, L., et al., J. Gen. Viro., 77 (Pt. 9), 2001-2008, (1996); and Liang, X., et al., Vaccine, 17, 2862-2872, (1999)). These studies indicate that the introduction of neutralizing antibodies against gp41 epitopes may be dependent on the native form of trimeric gp120-gp41 and that these epitopes may not be immunodominant, possibly due to the presence ofN-glycans. As shown in FIG. 2, the gp41 domain in the HIV-1 envelope glycoprotein contains four conserved glycosylation motifs (#22 (amino acids 608 of SEQ ID NO: 3), #23 (amino acids 613 of SEQ ID NO: 3), #24 (amino acids 622 of SEQ ID NO: 3), and #25 (amino acids 634 of SEQ ID NO: 3), and viruses with single or double mutations in these glycosylation sites replicated in both human and monkey T cell lines (Johnson, W.E., et al., J. Virol., 75, 11426-11436, (2001)). Removing the glycosylation motifs #24 (amino acid 622 of SEQ ID NO: 3) and #25 (amino acid 634 of SEQ ID NO: 3) near the neutralizing epitopes may increase the exposure of these epitopes and thus, enhance the induction of neutralizing antibodies against the gp41 domain (numbering glycosylation motifs from the N-terminus of the HIV 89.6 envelope glycoprotein).

It should also be note, that upon binding of the HIV envelope glycoprotein to its receptor molecules or the shedding of its surface subunit (gp120), its transmembrane subunit (gp41) converts into a six-helix bundle configuration, which is highly immunogenic but only present non-neutralizing epitopes (Kim, P.S., Annual Reviews of Biochemistry, 70, 777-810, (2001)). The use of a peptide containing the amino acid sequence corresponding to a segment of the gp41 (amino acid 629 to 664 of the SEQ ID NO: 1) can effectively block the transition into the undesirable six-helix bundle configuration. Therefore, treatment of VLPs with such a peptide can help to preserve the HIV envelope glycoprotein on the surface of the VLP to retain its native configuration for more efficient exposure of neutralizing epitopes and thus the induction of neutralizing antibodies. Such structural features are common to the envelope glycoproteins of many viral families, including, but not limited to, the envelope glycoproteins of retrovirus, influenza virus, and parainfluenza virus. Thus, such a VLP treatment approach can be applied to a variety of VLP vaccines.

In another example, the V1 loop, the V2 loop, and the V3 loop can be deleted to enhance the immunogenic properties of VLPs. Deletion of individual V1 or V2 loops does not reduce the potential of the virus to replicate in PBMCs or alter the co-receptor binding of the viral surface envelope glycoprotein (Stamatatos, L., et al., Aids Res. Hum. Retroviruses, 14, 1129-1139, (1998)). HIV-1 mutants lacking the V1 and V2 loops in gp120 exhibited increased sensitivity to neutralization by antibodies directed against V3 and a CD4-induced epitope on gp120, and by sera collected from patients infected with clades B, C, D, and F HIV-1 primary isolates (Cao, J., et al., J. Virol., 71, 9808-9812, (1997) and Stamatatos, L., and C. Cheng-Mayer, J. Virol. , 72, 7840-7845, (1998)). These studies suggest that the V2 loop or V2 together with the V1 loop shields some important neutralization epitopes with an overall structure that appears to be conserved among different HIV-1 primary isolates. Thus, deleting the V1-V2 loop or V2 loop may expose hidden neutralizing epitopes.

The V3 loop of the HIV envelope glycoprotein is highly variable and also constitutes a dominant epitope for the antibody response. Although neutralizing antibodies against this region are frequently detected, they are often strain-specific (Sattentau, Q.J., et al., Virol., 206(1), 713-7, (1995) and D'Souaza, M.P., et al., Aids, 9, 867-874, (1995)). Furthermore, deletion of the V3 loop has also been shown to increase the exposure of epitopes induced by sCD4 binding (Sanders, R.W., et al., J. Virol., 74, 5091-5100, (2000)). Lu et al. (Aids Res. Hum. Retroviruses, 14, 151-155, (1998)) compared antibody induction by gene-gun immunization of rabbits with DNA vectors expressing HIV-1 IIIB Gp160, Gp140, Gp120 and their corresponding V1/V2/V3 triple loop deletion mutants. These results showed that deletion of variable loops induced higher ELISA antibody responses but not neutralizing antibody responses.

In another study, Garrity et al. (J. Immunol., 159, 279-289, (1997)) showed that immunization of guinea pigs using recombinant vaccinia virus followed by protein boosting with mutant viral surface envelope glycoprotein 120, in which glycosylation sites were introduced to mask the immunodominant domain in the V3 loop, was more effective in inducing cross-reactive neutralizing antibodies against a divergent strain of the same subtype. Thus, eliminating the immunodominant epitopes in the V3 loop may enhance induction of cross-reactive antibodies. Furthermore, Kiszka et al. (J. Virol., 76, 4222-4232, (2002)) reported that immunization of mice using DNA vaccines encoding HIV envelope glycoproteins with V3 loop deletions induced broader cellular immune responses to subdominant epitopes and was more effective in conferring protection against challenge with recombinant vaccinia virus expressing heterologous HIV envelope glycoproteins, indicating that deletion of the V3 loop may also be advantageous in inducing broader cellular immune response.

In another embodiment, the RVFV envelope glycoprotein can include, but is not limited to, a RVFV GC envelope glycoprotein (SEQ ID NO: 4) and a RVFV GN envelope glycoprotein (SEQ ID NO: 5). The RVFV GC and GN envelope glycoproteins can be modified to enhance the immunogenic properties of the VLP 10. For example, the RVFV GC and GN envelope glycoproteins can be modified by truncating the cytoplasmic domain for the RVFV GC (amino acids 492-507 of SEQ ID NO: 4) and GN envelope glycoproteins (amino acids 458-527 of SEQ ID NO: 5).

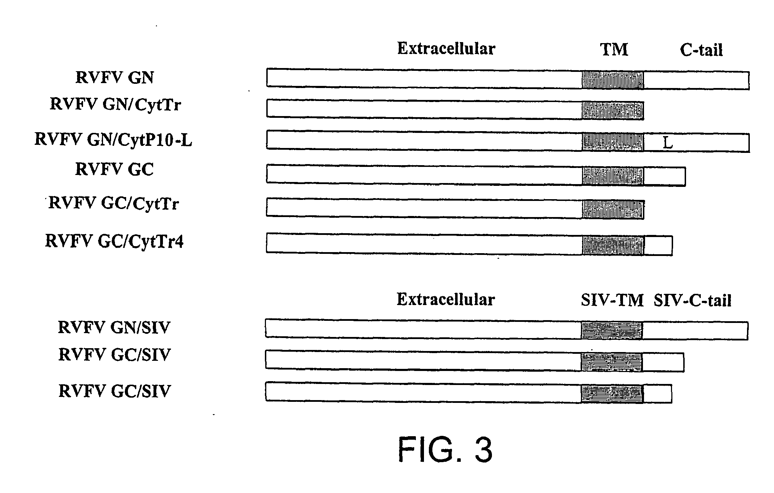

FIG. 3 illustrates some representative structural changes that can be made to the RVFV GN and GC envelope glycoproteins. For example, the RVFV GC and GN envelope glycoproteins can be modified by truncating the cytoplasmic domain for the RVFV GC (amino acids 492-507 of SEQ ID NO: 4) and GN envelope glycoproteins (amino acids 458-527 of SEQ ID NO: 5). In addition, the RVFV GN envelope glycoprotein can mutated by replacing the proline residue (amino acid 537 of SEQ ID NO: 5) from the cytoplasmic domain (conserved between RVFV and PTV GN envelope glycoprotein) with a leucine residue. Such modifications should increase levels of surface expression of the RVFV envelope glycoproteins and therefore increase their incorporation into VLPs. Thus, the effectiveness of the VLPs to elicit immune response against RVFV envelope glycoproteins may be enhanced since the VLPs may contain more RVFV envelope glycoproteins per unit amount.

Furthermore, the RVFV GC and GN envelope glycoproteins can be modified by replacing the transmembrane domain and/or the cytoplasmic tails of the RVFV GC and GN envelope glycoproteins with the transmembrane domain and the cytoplasmic tail of the SIV envelope glycoprotein. Studies on retrovirus assembly have shown that efficient incorporation of viral surface envelope glycoproteins may involve specific interaction between viral Gag proteins and the cytoplasmic domain of the viral surface envelope glycoprotein (Cosson, P., et al., EMBO J., 15, 5783-5788, (1996); Vincent, M.J., et al., J. Virol., 73, 8138-44, (1999); and Wyma, D.J., et al., J. Virol., 74, 9381-7, (2000)). Therefore, replacing the transmembrane domain and cytoplasmic tails of RVFV GN and GC envelope glycoproteins with those of the HIV or SIV envelope glycoprotein (SEQ ID NO: 6) or cytoplasmic tails (SEQ ID NO: 8) may produce chimeric proteins with increased cell surface expression and more efficient incorporation into SIV VLPs.

In previous studies, SIV envelope glycoproteins containing this truncated cytoplasmic domain yielded high levels of cell surface expression and more efficient incorporation into SIV VLPs in comparison to the SIV envelope glycoprotein with a full length cytoplasmic domain of over 150 amino acids (Vzorov, A. and Compans, R.W., J. Virol., 74, 8219-25, (2000)). Furthermore, the cytoplasmic domain of the SIV envelope glycoprotein contains a Tyr-based endocytosis signal, which has been shown to induce rapid endocytosis of the SIV envelope glycoprotein and lead to reduced surface expression (Labranche, C.C., et al., J. Virol., 69, 5217-5227, (1995)). Thus, attaching a truncated SIV envelope glycoprotein cytoplasmic domain to the RVFV envelope glycoproteins that are expressed on cell surface may produce enhanced VLPs.

In addition, Tyr residue can be replaced by Cys in the attached SIV cytoplasmic domain sequence (amino acid 16 of SEQ ID NO: 8) to further augment surface expression of designed chimeric proteins. This design for chimeric proteins can be applied to both RVFV GN and GC envelope glycoproteins. Such modifications may increase levels of surface expression of the RVFV envelope glycoproteins and therefore increase their incorporation into VLPs. Thus, the effectiveness of the VLPs to elicit immune response against RVFV envelope glycoproteins may be enhanced, since the VLPs contain more RVFV envelope glycoproteins per unit amount.

The adjuvant molecule 16, or at least a portion of the adjuvant molecule 16, is disposed (*e.g*., expressed) on the surface of the VLP 10. The adjuvant molecule 16 can interact with other molecules or cells (*e.g*., mucosal surfaces having sialic acid residues disposed thereon and antigen-presenting cells such as dendritic cells and follicular dendritic cells). The adjuvant molecule 16 can include, but is not limited to, an influenza hemagglutine (HA) adjuvant molecule (GenBank access number J02090), a parainfluenza hemagglutine-neuraminidase (HN) adjuvant molecule (GenBank access number z26523 for human parainfluenza virus type 3 HN sequence information), a Venezuelan equine encephalitis (VEE) adjuvant molecule (GenBank access number nc001449), a fins-like tyrosine kinase ligand (Flt3) adjuvant molecule (GenBank access number NM013520), a C3d adjuvant molecule (GenBank access number nm009778 for mouse C3 sequence and access number nm000064 for human C3 sequence), a mannose receptor adjuvant molecule, and a CD40 adjuvant molecule (GenBank access number m83312 for mouse CD40).

In general, the adjuvant molecule 16 sequence and the corresponding polynucleotide sequence can be found in GenBank and the access numbers can be obtained online at the NCBI. In addition, the sequences identified for the adjuvant molecules 16 above are only illustrative examples of representative adjuvant molecules 16. Further, variants that are substantially homologous to the above referenced adjuvant molecules 16 and adjuvant molecules 16 having conservative substitutions of the above referenced adjuvant molecules 16 can also be incorporated into VLPs 10 of the present invention to enhance the immunogenic characteristics of VLPs.

Mucosal immunity is critical for prevention of infection by aerosolized virus because mucosal cells can neutralize the virus and/or blocking virus attachment of the virus to the mucosal cells with secreted antibodies. However, little success has been documented for eliciting strong mucosal immune responses by non-replicating vaccines against viruses other than influenza, which is attributed, at least in part, to the difficulty of targeting the antigens to mucosal sites. In contrast, inactivated influenza virus has been shown to induce strong mucosal immune responses when administered mucosally, which may be the result of the strong binding affinity of the HA adjuvant molecule for sialic acid residues that are abundant at mucosal surfaces. Therefore, the affinity of HA adjuvant molecule for sialic acids may be utilized for targeting VLPs to mucosal surfaces. As discussed in more detail below, HA/SHIV VLPs are highly effective in eliciting strong mucosal immune responses against SHIV antigens when administered intranasally to mice. Thus, incorporating HA adjuvant molecules into VLPs may enhance the immunogenic properties of VLPs.

The following is a non-limiting illustrative example of an embodiment of the present invention that is described in more detail in (Guo et. al., "Enhancement of Mucosal Immune Responses by Phenotypically Mixed Influenza HA/SHIV Virus-Like Particles", (2003), in press). This example is not intended to limit the scope of any embodiment of the present invention, but rather is intended to provide specific experimental conditions and results. Therefore, one skilled in the art would understand that many experimental conditions can be modified, but it is intended that these modifications be within the scope of the various embodiments of this invention.

Mucosal immune responses against HIV play an important role in prevention of HIV infection and transmission, as the mucosal surface is the major site for initial HIV infection. Being the first line of defense, mucosal immunity is critical for prevention of infection by neutralizing virus and/or blocking virus attachment with secreted antibodies. However, little success has been documented for eliciting strong mucosal immune responses against HIV, which is attributed at least in part to the difficulty of targeting the antigens to mucosal sites. In contrast, inactivated influenza virus has been shown to induce strong mucosal immune responses when administered mucosally, a likely result of the strong binding affinity of its HA adjuvant molecule for sialic acid residues that are abundant on mucosal surfaces. Therefore, VLPs incorporating the HA adjuvant molecule may be suited to target mucosal surfaces since the HA adjuvant molecule has an affinity for sialic acid.

To enhance mucosal immune responses using SHIV VLPs as a mucosal AIDS vaccine, phenotypically mixed influenza HA/SHIV virus-like particles (HA/SHIV 89.6 VLPs) were produced and used to intranasally immunize C57B/6J mice. Production of phenotypically mixed HA/SHIV 89.6 VLPs, which possess both biologically active HIV envelope glycoproteins and influenza HA adjuvant molecules, may be important in elicitation of enhanced immune responses against HIV envelope proteins. Therefore, baculovirus-derived SHIV 89.6 VLPs and HA/SHIV 89.6 VLPs were produced through co-infection of insect cells with rBV SIV Gag, rBV HIV envelope glycoprotein, both with and without rBV HA adjuvant molecule.

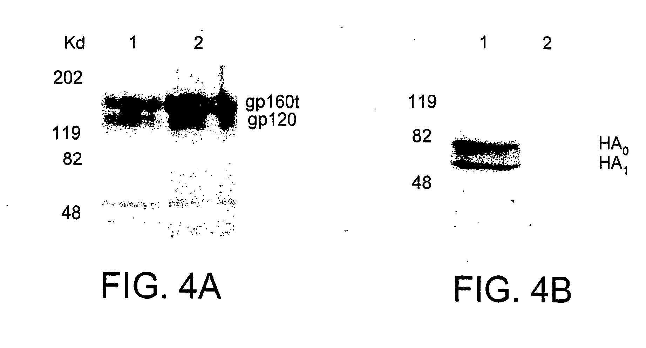

FIGS. 4A and 4B illustrate that both HIV envelope glycoproteins and influenza HA adjuvant molecules can be detected in HA/SHIV 89.6 VLPs by using Western Blot analysis blotting with antibody against HIV envelope glycoproteins (FIG. 4A) or influenza HA adjuvant molecules (FIG. 4B), respectively. Both HIV envelope glycoprotein and influenza HA adjuvant molecule are partially cleaved into their active state gp120 and HA1. In addition, the ability of chimeric VLPs to induce hemagglutination (HA) was examined, a functional property of influenza HA. HA titers of chimeric VLPs were determined by incubating equal volumes of serial two-fold dilutions of HA/SHIV VLPs in PBS-def (PBS deficient in Mg²⁺ and Ca²⁺) with chicken red blood cells (final concentration 0.5%) for 1 hour (h) at room temperature. The HA titer of HA/SHIV 89.6 chimeric VLPs were found to be as high as 1:4000, whereas SHIV 89.6 VLPs showed negative in HA titer.

FIGS. 5A through 5B illustrate graphs measuring various characteristics of a number of VLPs that were intranasally introduced into mice. In FIGS. 5A through 5D, "V" represents SHIV VLPs, "HA/V" represents HA/SHIV VLPs, "V+CT" represents SHIV VLPs (10 µg) + cholera toxin (CT) (10 µg), and "N" represents a negative control (PBS). In addition, the number in parentheses indicates the "µg's" of VLPs used for immunization of the mice.

FIG. 5A is a graph measuring serum IgG levels specific to HIV envelope glycoproteins. FIG. 5B is a graph measuring splenocytes producing IFN-γ determined by ELISPOT assay. FIG. 5C is a graph measuring HIV envelope glycoprotein-specific IgA in vaginal wash. FIG. 5D is a graph measuring HIV envelope glycoprotein-specific IgA in fecal extracts.

In particular, systemic and mucosal antibody responses, as well as cytotoxic T cell (CTL) responses, of mice immunized with SHIV 89.6 VLPs or HA/SHIV 89.6 VLPs are shown in FIGS. 5A through 5D. Intranasal immunizations were given with VLPs either with or without addition of CT. The level of serum IgG production to HIV envelope glycoprotein was found to be highest in the group immunized with phenotypically mixed HA/SHIV 89.6 VLPs. Similarly, mucosal IgA production was also found to be enhanced in the group immunized with HA/SHIV VLP mucosally. Analysis of the IgG1/IgG2a ratio indicated that a Th1-oriented immune response resulted from these VLPs immunizations. HA/SHIV VLP-immunized mice also showed significantly higher CTL responses than those observed in SHIV VLP-immunized mice. Furthermore, a MHC class I restricted T cell activation ELISPOT assay showed elevated IFN-γ, IL-2, and IL-12 production in HA/SHIV VLP-immunized mice, indicating that phenotypically mixed HA/SHIV VLPs can enhance both humoral and cellular immune responses at multiple mucosal sites. Thus, a heterologous adjuvant molecule, the HA adjuvant molecule, can be coexpressed with retrovirus proteins in infected cells resulting in its efficient incorporation into retroviral VLPs in a biologically active form. In addition, the resultant VLPs exhibit enhanced immunogenicity, especially when delivered by a mucosal route. These results demonstrate the feasibility of construction of a range of VLPs containing heterologous surface molecules for possible use as improved vaccine antigens. Therefore, chimeric HA-containing VLPs may be used for the mucosal immunization against HIV.

The possibility of pre-existing immunity to the influenza HA protein is a factor that should be considered in the evaluation of HA-VLP vaccines. However, it is uncertain that the existence of such preexisting immunity would negatively impact the immune responses to the VLPs (in marked contrast to immunization using replicating vectors). It is in fact possible that preexisting antibodies would lead to production of immune complexes would enhance targeting of VLPs to follicular dendritic cells and thus result in stimulation of B cell responses.

Nevertheless, alternative approaches can be applied for mucosal targeting of VLPs. One possibility is to utilize influenza HA adjuvant molecules from other influenza virus species to which there is no preexisting immunity in the human population. There are about 15 such non-cross reactive serotypes of HA adjuvant molecules, which have been identified, which are antigenically non-overlapping based on tests with polyclonal immune sera. The 15 non-cross reactive serotypes of HA viruses and their replication are described in the following publications: Lamb, R.A. and Krug, R.M., Orthomyxoviridae, (1996) and Fields, B.N., et. al., Editors, Field's Virology, Lippincott-Raven Publishers, Philadelphia, PA, 1353-1395, (1996). Thus, VLPs can be produced containing one or more of these alternative HA adjuvant molecule subtypes, therefore avoiding a possible affect of preexisting anti-HA immunity on induction of immune responses against VLP antigens. It should be noted that some influenza HA adjuvant molecules from other species may bind preferentially to sialic acid linkages not found on human cells. This property, however, can be modified by mutation of specific HA amino acids (Vines, A., et al., J. Virol., 72, 7626-7631, (1998)).

An alternative approach to using HA adjuvant molecules is the production of VLPs containing parainfluenza virus HN adjuvant molecules. Like HA adjuvant molecules, the HN adjuvant molecules attach specifically to the sialic acid residues at mucosal surfaces. Thereore, chimeric VLPs containing HN adjuvant molecules should have similar mucosal targeting properties as the HA adjuvant molecules. However, immune responses to the proteins of human parainfluenza viruses are of relatively short duration, and reinfections with the same viral serotypes are known to occur (Glezen, W.P., et al., J. Infect. Dis., 150, 851-857, (1984)). Thus, as compared with HA, it is less likely that preexisting immunity to the HN adjuvant molecules of a parainfluenza virus would affect mucosal delivery of a VLP vaccine.

HN-VLPs may be easier to produce in modified vaccinia Ankara expression systems rather than baculovirus expression systems. This is because the release of HA chimeric VLPs from mammalian cells would require addition of exogenous neuraminidase (Bosch, V., et al., J. Gen. Virol., 82, 2485-2494, (2001)) since sialic acid would be added to the envelope glycoproteins as a terminal sugar and lead to aggregation of VLPs at the cell surface (which does not occur in the insect cell-produced VLPs). In contrast, HN carries its own neuraminidase. Studies of viral pseudotypes have shown that the glycoproteins of parainfluenza viruses including the HN adjuvant molecule can be assembled into virions of retroviruses, indicating that this type II membrane protein can be incorporated into VLPs (Spiegel, M. et al., J. Virol., 72, 5296-5302, (1998)). Thus, incorporating HN adjuvant molecules into VLPs may enhance the immunogenic properties of VLPs.

Antigen presenting cells can be targeted by VLPs by including one or more of the following adjuvant molecules on the surface of the VLP: the VEE adjuvant molecule, the Flt3 adjuvant molecule, the mannose adjuvant molecule, the CD40 adjuvant molecule, and the Cd3 adjuvant molecule. In particular, the VEE adjuvant molecule, the Flt3 adjuvant molecule, the mannose adjuvant molecule, and the CD40 adjuvant molecule can be used to target dendritic cells, while the Cd3 adjuvant molecule can be used to target follicular dendritic cells.

Dendritic cells (DCs) are very efficient antigen presenting cells involved in priming native CD4 and CD8 T cells, thus inducing primary immune responses and permitting establishment of immunological memory (Inaba, K., et al., J. Exp. Med., 166:182-194, (1987) and Inaba, K., et al., J. Exp. Med., 172:631-640, (1990)). Antigens taken up by DCs are expressed at the cell surface in the form of peptides associated with MHC class II, which stimulates CD4 Th cells. For induction of CD8 T cells, MHC class 1 associated peptides are derived from endogenously synthesized proteins as well as from some exogenous antigens (*e.g*., infectious agents, dying cells, proteins associated with inert particles, and immune complexes) by DC endocytosis (Heath, W.R. and F. R. Carbone, Curr. Opin. Immunol., 11:314-318, (1999); Reimann, J. and R. Schirmbeck, Immunol. Rev., 172; 131-152, (1999); Regnault, A., et al., J. Exp. Med., 189:371-380, (1999); and Machy, P., et al., Eur. J. Immunol., 30:848-857, (2000)). DCs harboring immune complexes also stimulate naive B cells (Wykes, M., J. Immunol., 161,1313-1319, (1998) and Dubois, B., et al., Biol., 70, 633-641, (2001)). The highly developed Ag-presenting capacity of DCs has led to their study of cellular vaccine adjuvants for the immunotherapy of cancer (Schuler, G. and R.M. Steinman, J. Exp. Med., 1986: 1183-1187, (1997) and Baggers, J., et al., J. Clin. Oncol., 18:3879-3882, (2000)). HIV and SIV virions interact with DCs via DC-SIGN and/or CD4 receptors; however, this interaction appears to preferentially result in infection of the DCs as well as transmission to other target cells rather than potentiation of an immune response (Geijtenbeek, T.B., et al., Cell, 100: 587-597, (2000) and Geijtenbeek, T.B., et al., Immunol. Lett. 79:101-107, (2001)). On the other hand, inert particulate antigens like VLPs are very attractive target for antigen presenting cells, particularly DCs (Bachmann, M.F., et al., Eur. J. Immunol., 26:2595-2600, (1996); Ruedl, C., et al., Eur. J. Immunol., 32:818-825, (2002) and Da Silva, D.M., et al., Int. Immunol., 13:633-641, (2001)). Therefore, the interaction of VLPs with DCs may result in potentiating DCs to initiate T cell activation.

The possible advantage of targeting vaccine antigens to DCs is indicated by the extremely small number of DCs in peripheral tissues and in blood, where DCs represent less than 1 % of total cell number. Flt3 ligand (FL) adjuvant molecule (GenBank access number NM013520) is a hematopoietic growth factor that has the unique ability to expand the number of both CD8α- and CD8α+ DC subsets (Lyman, S.D., et al., Cell, 75:1157-1167, (1993); Maraskovsky, E, et al., J. Exp. Med., 184:1953-1962, (1996); Maraskovsky, E, et al., Blood, 96:878-884, (2000) and Pulendran, B., et al., J. Immunol., 159:222-2231, (1997)). Such expansion of DCs in mice resulted in dramatic increases in Ag-specific B and T cell responses (Pulendran, B., et al., J. Exp. Med., 188, 2075-2082, (1998)), enhanced T-cell mediated immune responses (Pisarev et al., Int J Immunopharmacol, 11, 865-76, (2000)), and protective immunity to Listeria monocytogenes (Gregory, S.H., et al., Cytokine, 13:202-208, (2001)). It is suggested that FL treatment increases the capacity of DCs as antigen presenting cells by up-regulating MHC and costimulatory molecules (CD40, CD86), and by inducing production of cytokines (IFN-y, IL-2, IL-12 or IL-4) (Pulendran, B., et al., J. Exp. Med., 188, 2075-2082, (1998) and Pulendran, B., et al., Proc. Natl. Acad. Sci., U.S.A. 96, 1036-1041, (1999)). Therefore, incorporation of FL adjuvant molecules into VLPs may enhance the immunogenic properties of the VLPs.

The VLP can be produced to include the FL adjuvant molecule by PCR-amplifying and cloning the whole mouse FL gene including the signal sequence and transmembrane (TM) domain into rBV transfer vector pc/pS1. To construct a rBV expressing FL, Sf9 insect cells can be co-transfected with Baculogold DNA (available from PharMingen, Inc.) and the pc/pS 1 transfer vector containing the FL gene.

The incorporation of the FL adjuvant molecule into VLPs can be enhanced by modifiying the FL adjuvant molecule. In particular, the extracellular coding domain of the FL gene (from the end of signal peptide to the start of the TM segment) (SEQ ID NO: 7) can be fused to the N-terminus of the SIV Env glycoprotein-41 TM domain (SEQ ID NO: 6) and the tPA signal peptide can be fused to the N-terminus of the FL-chimeric protein (SEQ ID NO: 9). An alternative approach is to produce a glycosyl-phosphatidylinositol (GPI)-anchored form the FL adjuvant molecule (designated as FL-GPI) using a pcDNA3-GPt cassette (GenBank access number x52645), which was previously used to produce GM-CSF in an active membrane-bound form (Poloso, N.J., et al., Mol. Immunol., 38:803-816, (2002)). GPI-anchored proteins preferentially associate with lipid rafts, which are used as sites for virus assembly (Nguyen, D. H. and J.E. Hildreth; J. Virol., 74:3265-3272, (2000)). These chimeric FL constructs can be cloned into pc/pS1 and used to produce rBVs expressing FL fusion proteins.

VEE is a member of the family Togaviridae and is typically transmitted by mosquitoes to humans or other animals, in which it causes fever and encephalitis. Following inoculation into the footpad of mice, the virus was observed to be rapidly transported to the draining lymph nodes. Recent studies have shown that dendritic cells in the lymph nodes are the primary target of VEE infection, and VEE replicon particles were observed to be localized in Langerhans cells, dendritic cells of the skin, following subcutaneous inoculation (Macdonald, G. H., and Johnston, R.E., J Virol., 74(2), 914-22, (2000)). These investigators also showed that the targeting of VEE adjuvant molecules to DCs was dependent upon the specific amino acid sequence of the viral envelope glycoprotein E2. Therefore, VLPs incorporating VEE adjuvant molecules may be used to target dendritic cells.

Dendritic cells use the mannose receptor (MR) as the major receptor for endocytosis of antigens (Sallusto, F., et al., J. Exp. Med., 192(2), 389-400, (1995)). This receptor is a 175 kD protein containing eight carbohydrate recognition domains with high affinity for mannose-rich glycoproteins (Stahl, P.D., Curr Opin Immunol., 4(1), 49-52, (1992) and Ezekowitz, R.A., et al., J Exp. Med., 172(6), 1785-94, (1990)). Following endocytosis, the MR releases its ligand at low pH and it recycles to the cell surface, thus having the capacity to interact with ligands in multiple rounds (Stahl, P., et al.; Cell, 19(1), 207-15, (1980)). It has been suggested that the MR may provide a mechanism for distinguishing self from non-self antigens on the basis of glycosylation patterns since, in higher eukaryotes, mannose residues are usually buried within the carbohydrate moieties of evelope glycoproteins and therefore not available for binding to MR (Sallusto, F., et al., J. Exp. Med. 192(2), 389-400, (1995)). Thus, it may be possible to target VLPs to dendritic cells on the basis of distinct oligosaccharide profiles.

Once dendritic cells take up antigens, immature dendritic cells need to differentiate into professional antigen presenting cells in response to maturation signals. As dendritic cells mature, expression of co-stimulatory molecules and MHC-peptide complexes increase and cytokines are produced (Banchereau, J. &I Steinman, R.M., Nature, 392, 245-52, (1998) and Pierre, P., Turley, et al., Nature, 388, 787-92, (1997)). Interaction between CD40 expressed on antigen presenting cells including dendritic cells and CD40L on activated Th cells is important for T cell dependent B cell activation and isotype switching (Rousset, F., et al., J. Exp. Med., 173,705-10, (1991)). CD40 ligation with a cell line expressing CD40L activates Langerhans cell-derived dendritic cells, and induces high level expression of MHC II and accessory molecules such as CD80 and CD86 (Caux, C., et al., J. Exp. Med., 180, 1263-1272, (1994)). Cross-linking CD40 with anti-CD40 antibodies play a role in ablating the tolerogenic potential of lymphoid dendritic cells (Grohmann U., et al., J. Immunol. 166, 277-83, (2001)). It is also shown that signaling through CD40 on the antigen presenting cells can replace the requirement for "help" from CD4 Th cells in inducing CTL activities (Bennett, S.R., et al., Nature, 393, 478-480, 1993 and Schoenberger, S.P., et al., Nature, 393, 480-483, (1998)). In anti-tumor pre-clinical model studies, it is indicated that the main mediator for dendritic cell activation is CD40 receptor engagement (Ribas, A., et al., Cancer Res., 61, 8787-8793, (2001) and Ridge, J.P., et al., Nature, 393,474-478, (1998)). These studies suggest that CD40L seem to provide important maturation signals for dendritic cells. Therefore, VLPs incorporating CD40L adjuvant molecules may be used to target dendritic cells.

Follicular dendritic cells (FDCs) play an important role in germinal centers, where antibody-forming cells are generated. Recent studies have indicated that FDCs play an important co-stimulatory role in the enhancement of antibody responses (Qin, D., et al. J. Immunol., 161, 4549-4554, (1998); Fearon, D. T. and Carroll, M.C.; Annu. Rev. Immunol., 18, 393-422, (2000); Father, M., et al., Eur. J. Immunol., 31, 176-185, (2001) and Tew, J.G., et al., Trends Immunol., 22, 361-367, (2001)). During HIV infection, immune complexes containing virions are found in association with FDCs (Hlavacek, W.S., et al.; Philos. Trans. R. Soc. Lond B Biol. Sci., 355, 1051-1058, (2000); Rosenberg, Y.J., et al., Dev. Immunol., 6, 61-70, (1998); Smith, B.A. et al.; J. Immunol., 166, 690-696, (2001)), and such complexes could play a significant role in effective antigen presentation to B cells for induction of neutralizing antibody as observed during HIV infection *in vivo.* Because of their close similarity to virions, VLPs may mimic such immune complexes much more closely than soluble antigens.

The FDCs interact with components of the complement system including C3d, and it was recently demonstrated that recombinant proteins containing a segment of the C3d adjuvant molecule fused (amino acids 1024 to 1320 of SEQ ID NO: 11) to an antigen resulted in a striking increase in the efficiency of the antibody response (Dempsey, P.W., et al., Science, 271, 348-350, (1996)). Complement is a plasma protein system of innate immunity that is activated by microorganisms in the absence of antibody (Fearon, D.T. and Austen, K.F., N. Engl. J. Med., 303, 259-263, (1980)). Upon activation, C3d fragment binds to its receptor, CR2 (CD21) which is primarily expressed on B cells and FDCs (Fearon, D. T. and Carter, R. H.; Annu. Rev. Immunol., 13:17-149, (1995)). The presence of C3d adjuvant molecules on the surfaces of the VLPs may result in their enhanced interaction with FDCs and B cells, and thus stimulation of the antibody responses to viral surface envelope glycoproteins contained in the VLP structure.

Because of the relatively large size of the C3d adjuvant molecule fragment, which is about 300 amino acids in length, two factors that may affect its function: 1) its proper exposure for interaction with CR2 on FDC; and 2) its potential interference with the proper folding of the protein antigen. Two alternative approaches can be used to incorporate the C3d fragment into VLPs in order to elucidate antibody responses against viral surface glycoproteins incorporated into the VLPs.

First, the C3d adjuvant molecule fragment (amino acids 1024 to 1320 of SEQ ID NO: 11) can be fused to the N-terminus of the selected viral surface envelope glycoprotein and the tPA signal peptide (SEQ ID NO: 9) can be introduced at the N-terminus of the viral surface envelope glycoprotein. Second, the tPA signal peptide can be fused to the N-terminus of C3d adjuvant molecule and a membrane anchoring sequence (TM domain of viral glycoproteins, example SIV envelope TM (SEQ ID NO: 6) or the GPI-anchoring sequence (GenBank access number x52645, SEQ ID NO: 10)) can be fused to the C-terminus of the C3d adjuvant molecule.

VLPs can be produced by *in vitro* cell culture expression systems such as, but not limited to, recombinant baculovirus expression system (BEVS) (Yamshchikov, G. V., Ritter, G. D., Vey, M., and Compans, R. W. Virology, 214, 50-58, (1995). Assembly of SIV virus-like particles containing envelope proteins can be performed using expression systems, such as, but not limited to, a baculovirus expression system (Yamshchikov, G. V., Ritter, G. D., Vey, M., and Compans, R. W., Virology, 214, 50-58, (1995)), recombinant modified vaccinia Ankara expression system (MVA) (Wyatt LS, et. al., Vaccine, 15, 1451-8, (1996)), recombinant VSV, recombinant adenovirus, and recombinant DNA expression vectors. Preferably, the VLPs are produced using recombinant BEVS and recombinant MVA expression systems.

In general, VLPs can be produced by simultaneously introducing into a cell a viral protein expression vector, a viral surface envelope glycoprotein expression vector, and/or an adjuvant molecule expression vector. The expressed viral protein self-assembles into a VLP that incorporates the viral surface envelope glycoprotein and/or the adjuvant molecule. The viral surface envelope glycoprotein and/or the adjuvant molecule are expressed on the VLP surface. Thereafter, the cell produces the VLP (*e.g*., chimeric and/or phenotypically mixed VLP). The cells can include, but are not limited to, insect cells (*e.g., spodopera frugiperda* Sf 9 cells and Sf21cells) and mammalian cells (*e.g*., EL4 cells and HeLa cells).

In general, the viral protein expression vector can be produced by operably linking a coding sequence for a viral protein of a virus to an appropriate promoter (*e.g*., an early promoter, late promoter, or hybrid late/very late promoter). The viral protein expression vector can also be modified to form a viral protein expression construct. In addition, the viral surface envelope glycoprotein expression vector can be produced by operably linking a coding sequence for a viral surface envelope glycoprotein of a virus to an appropriate promoter (*e*.*g*., early promoter, late promoter, or hybrid late/very late promoter). The viral surface envelope glycoprotein expression vector can be modified to form a viral surface envelope glycoprotein expression construct. Similarly, the adjuvant molecule expression vector can be produced by operably linking a coding sequence for an adjuvant molecule to an appropriate promoter (*e.g*., early promoter, late promoter, or hybrid late/very late promoter). The adjuvant molecule expression vector can be modified to form an adjuvant molecule expression construct.

In the case of where the adjuvant molecule is mannose, the adjuvant molecular expression construct is not needed because the mannose molecules can be chemically added to VLPs after the VLPs are produced.

The term "host" includes humans, mammals (*e.g*., cats, dogs, horses, and cattle), and other living species that are in need of treatment. Hosts that are "predisposed to" condition(s) can be defined as hosts that do not exhibit overt symptoms of one or more of these conditions but that are genetically, physiologically, or otherwise at risk of developing one or more of these conditions.

The term "treat", "treating", and "treatment" are an approach for obtaining beneficial or desired clinical results. For purposes of embodiments of this invention, beneficial or desired clinical results include, but are not limited to, alleviation of symptoms, diminishment of extent of disease, stabilization (*i.e*., not worsening) of disease, preventing spread of disease, delaying or slowing of disease progression, amelioration or palliation of the disease state, and remission (partial or total) whether detectable or undetectable. In addition, "treat", "treating", and "treatment" can also mean prolonging survival as compared to expected survival if not receiving treatment.

The term "condition" and "conditions" denote a state of health that can be related to infection by a virus. The infections that are discussed herein are to be included as conditions that can be treated by embodiments of the present invention.

"Polypeptide" refers to peptides, proteins, glycoproteins, and the like, of the present invention comprising two or more amino acids joined to each other by peptide bonds or modified peptide bonds, (*i.e*., peptide isosteres). "Polypeptide" refers to both short chains, commonly referred to as peptides, oligopeptides, or oligomers, and to longer chains, generally referred to as proteins. "Polypeptides" may contain amino acids other than the 20 gene-encoded amino acids. "Polypeptides" include amino acid sequences modified either by natural processes, such as post-translational processing, or by chemical modification techniques, which are well known in the art. Such modifications are described in basic texts and in more detailed monographs, as well as in a voluminous research literature.

Modifications may occur anywhere in the polypeptides, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched, and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (Proteins-Structure and Molecular Properties, 2nd Ed., T. E. Creighton, W. H. Freeman and Company, New York, 1993; Wold, F., Post-translational Protein Modifications: Perspectives and Prospects, pgs. 1-12 in Post-translational Covalent Modification of Proteins, B. C. Johnson, Ed., Academic Press, New York, 1983; Seifter, et al., Meth. Enzymol., 182: 626-646, (1990), and Rattan, et al., Ann NY Acad. Sci., 663:48-62, (1992)).

"Variant" refers to polypeptides that differ from a reference polynucleotide or polypeptide, but retains essential properties. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, and deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including, but not limited to, those described in Computational Molecular Biology, Lesk, A. M., Ed., Oxford University Press, New York, (1988); Biocomputing: Informatics and Genome Projects, Smith, D. W., Ed., Academic Press, New York, (1993); Computer Analysis of Sequence Data, Part I, Griffin, A. M., and Griffin, H. G., Eds., Humana Press, New Jersey, (1994); Sequence Analysis in Molecular Biology, von Heinje, G., Academic Press, 1987; and Sequence Analysis Primer, Gribskov, M. and Devereux, J., Eds., M Stockton Press, New York, (1991); and Carillo, H., and Lipman, D., SIAM J Applied Math., 48, 1073 (1988).

Preferred methods to determine identity are designed to give the largest match between the sequences tested. Methods to determine identity and similarity are codified in publicly available computer programs. The percent identity between two sequences can be determined by using analysis software (*e*.*g*., Sequence Analysis Software Package of the Genetics Computer Group, Madison Wis.) that incorporates the Needelman and Wunsch, (J. Mol. Biol., 48, 443-453, (1970)) algorithm (*e.g*., NBLAST, and XBLAST). The default parameters are used to determine the identity for the polynucleotides and polypeptides.

By way of example, the polypeptide sequences may be identical to one or more of the reference sequences described above, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the reference polypeptide by the numerical percent of the respective percent identity (divided by 100) and then subtracting that product from said total number of amino acids in the reference polypeptide.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "substantially homologous" is used herein to denote polypeptides having about 50%, about 60%, about 70%, about 80%, about 90%, and preferably about 95% sequence identity to the sequences discussed above. Percent sequence identity is determined by conventional methods as discussed above.

In general, homologous polypeptides are characterized as having one or more amino acid substitutions, deletions, and/or additions. These changes are preferably of a minor nature (*e*.*g*., conservative amino acid substitutions and other substitutions that do not significantly affect the activity of the polypeptide).

In addition, embodiments include polynucleotides that encode polypeptides having one or more "conservative amino acid substitutions" of the sequences discussed above. Conservative amino acid substitutions can be based upon the chemical properties of the amino acids. Variants can be obtained that contain one or more amino acid substitutions of the sequences discussed above, in which an alkyl amino acid is substituted for an alkyl amino acid in a polypeptide, an aromatic amino acid is substituted for an aromatic amino acid in a polypeptide, a sulfur-containing amino acid is substituted for a sulfur-containing amino acid in a polypeptide, a hydroxy-containing amino acid is substituted for a hydroxy-containing amino acid in a polypeptide, an acidic amino acid is substituted for an acidic amino acid in a polypeptide, a basic amino acid is substituted for a basic amino acid in a polypeptide, or a dibasic monocarboxylic amino acid is substituted for a dibasic monocarboxylic amino acid in a polypeptide.

Among the common amino acids, for example, a "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine. Other conservative amino acid substitutions include amino acids having characteristics such as a basic pH (arginine, lysine, and histidine), an acidic pH (glutamic acid and aspartic acid), polar (glutamine and asparagine), hydrophobic (leucine, isoleucine, and valine), aromatic (phenylalanine, tryptophan, and tyrosine), and small (glycine, alanine, serine, threonine, and methionine).

Polypeptides having conservative amino acid variants can also comprise non-naturally occurring amino acid residues. Non-naturally occurring amino acids include, without limitation, trans-3-methylproline, 2-4-methanoproline, cis-4-hydroxyproline, trans-4-hydroxyproline, N-methyl-glycine, allo-threonine, methylthreonine, hydroxyethylcysteine, hydroxyethylhomocysteine, nitro-glutamine, homoglutamine, pipecolic acid, thiazolidine carboxylic acid, dehydroproline, 3- and 4-methylproline, 3,3-dimethylproline, tert-leucine, norvaline, 2-azaphenyl-alanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations are carried out in a cell-free system comprising an *E*. *coli* S30 extract and commercially available enzymes and other reagents. Proteins are purified by chromatography. (Robertson, et al., J. Am. Chem. Soc., 113, 2722, (1991); Ellman, et al., Methods Enzymol., 202, 301, (1991); Chung, et al., Science, 259, 806-9, (1993); and Chung, et al., Proc. Natl. Acad. Sci. USA, 90, 10145-9, (1993)).

In a second method, translation is carried out in Xenopus oocytes by microinjection of mutated mRNA and chemically aminoacylated suppressor tRNAs (Turcatti, et al., J. Biol. Chem., 271, 19991-8, (1996)). Within a third method, *E*. *coli* cells are cultured in the absence of a natural amino acid that is to be replaced (*e.g*., phenylalanine) and in the presence of the desired non-naturally occurring amino acid(s) (*e.g*., 2-azaphenylalanine, 3-azaphenylalanine, 4-azaphenylalanine, or 4-fluorophenylalanine). The non-naturally occurring amino acid is incorporated into the protein in place of its natural counterpart. (Koide, et al., Biochem., 33, 7470-6, (1994)). Naturally occurring amino acid residues can be converted to non-naturally occurring species by *in vitro* chemical modification. Chemical modification can be combined with site-directed mutagenesis to further expand the range of substitutions (Wynn, et al., Protein Sci., 2, 395-403, (1993)).

A "chimeric" VLP, as used herein, can be defined as a VLP having at least viral surface envelope glycoprotein incorporated into the VLP, wherein the viral protein and the viral surface envelope glycoprotein are from different viruses.

A "phenotypically mixed" VLP, as used herein, can be defined can be defined as a VLP having at least one adjuvant molecule incorporated into the VLP, wherein the viral protein and the adjuvant molecule are from different viruses.

"Expressed", as used herein, can be defined as being a molecule disposed, or a portion of the molecule disposed, upon the surface of the VLP.

An "expression construct" is an expression vector containing a coding sequence for a recombinant protein.

The term "recombinant" when used with reference to a cell, or nucleic acid, or vector, indicates that the cell, or nucleic acid, or vector, has been modified by the introduction of a heterologous nucleic acid or the alteration of a native nucleic acid, or that the cell is derived from a cell so modified. Thus, for example, recombinant cells express genes that are not found within the native (non-recombinant) form of the cell or express native genes that are otherwise abnormally expressed, under-expressed or not expressed at all.

The term "operably linked" refers to the arrangement of various nucleotide sequences relative to each other such that the elements are functionally connected to and are able to interact with each other. Such elements may include, without limitation, one or more promoters, enhancers, polyadenylation sequences, and transgenes. The nucleotide sequence elements, when properly oriented, or operably linked, act together to modulate the activity of one another, and ultimately may affect the level of expression of the transgene. The position of each element relative to other elements may be expressed in terms of the 5' terminus and the 3' terminus of each element, and the distance between any particular elements may be referenced by the number of intervening nucleotides, or base pairs, between the elements.

A "vector" is a genetic unit (or replicon) to which or into which other DNA segments can be incorporated to effect replication, and optionally, expression of the attached segment. Examples include plasmids, cosmids, viruses, chromosomes and minichromosomes. Particularly preferred expression vectors (for expression of an attached segment) are baculovirus vectors and modified vaccinia Ankara vectors.

A "coding sequence" is a nucleotide sequence that is transcribed into mRNA and translated into a protein, *in vivo* or *in vitro.*

"Regulatory sequences" are nucleotide sequences, which control transcription and/or translation of the coding sequences, which they flank.

"Processing sites" are described in terms of nucleotide or amino acid sequences (in context of a coding sequence or a polypeptide). A processing site in a polypeptide or nascent peptide is where proteolytic cleavage occurs, where glycosylation is incorporated or where lipid groups (such as myristoylation) occurs. Proteolytic processing sites are where proteases act.

"Virus-like particles" (VLPs) are membrane-surrounded structures comprising viral envelope proteins expressed on the VLP. In addition, adjuvant molecules can be expressed on the VLP. Further, viral core proteins are located within the membrane of the VLP. Additional components of VLPs, as known in the art, can be included within or disposed on the VLP. VLPs do not contain intact viral nucleic acids, and they are non-infectious. Desirably, there is sufficient viral surface envelope glycoprotein and/or adjuvant molecules expressed, at least in part, on the surface of the VLP so that when a VLP preparation is formulated into an immunogenic composition and administered to an animal or human, an immune response (cell-mediated or humoral) is raised.

A "truncated" viral surface envelope glycoproteins are ones which contain less than a full length cytoplasmic domain, which retains surface antigenic determinants against which an immune response is generated, preferably a protective immune response, and it retains sufficient envelope sequence for proper precursor processing and membrane insertion. The skilled artisan can produce truncated virus envelope proteins using recombinant DNA technology and virus coding sequences, which are readily available to the public.

Truncation of the viral surface envelope protein is from the carboxy terminus of the viral surface envelope glycoprotein. Contemplated are truncated viral surface envelope glycoproteins retaining from about 0% to about 90% of the cytoplasmic domain, where the cytoplasmic domain for a type I viral surface envelope glycoprotein is the region between the C-terminus of the transmembrane domain and the C-terminus of the entire envelope protein. Preferably from about 10% to 25% of the cytoplasmic domain is retained. Also encompassed by "truncated," are all percentages and ranges, within the 5% to 90% range, including from about 15% to about 50%.

VLPs based on cloned viral surface envelope glycoproteins, and preferably further comprising Gag proteins from the same viruses, can be readily produced without the expense of undue experimentation by the ordinary skilled artisan using the teachings of the present application taken with vectors as described herein and what is well known to and readily accessible in the art.

In another embodiment, polyclonal and/or monoclonal antibodies capable of specifically binding to the VLP are provided. The term "antibody" is used to refer both to a homogenous molecular entity, or a mixture such as a serum product made up of a plurality of different molecular entities. Monoclonal or polyclonal antibodies, which specifically react with the VLPs of the present invention, may be made by methods known in the art. (*e*.*g*., Harlow and Lane (1988) Antibodies: A Laboratory Manual, Cold Spring Harbor Laboratories; Goding (1986) Monoclonal Antibodies: Principles and Practice, 2d ed., Academic Press, New York; and Ausubel *et al.* (1987)). Also, recombinant immunoglobulins may be produced by methods known in the art, including but not limited to, the methods described in U.S. Pat. No. 4,816,567.

Antibodies specific for VLPs and viral surface envelope glycoproteins of viruses may be useful, for example, as probes for screening DNA expression libraries or for detecting the presence of the cognate virus in a test sample. Frequently, the polypeptides and antibodies will be labeled by joining, either covalently or noncovalently, a substance that provides a detectable signal. Suitable labels include but are not limited to radionuclides, enzymes, substrates, cofactors, inhibitors, fluorescent agents, chemiluminescent agents, magnetic particles and the like. United States Patents describing the use of such labels include but are not limited to U.S. Pat. Nos. 3,817,837; 3,850,752; 3,939,350; 3,996,345; 4,277,437; 4,275,149; and 4,366,241.

Antibodies specific for VLPs and retroviral surface envelope glycoproteins may be useful in treating animals, including humans, suffering from cognate viral disease. Such antibodies can be obtained by the methods described above and subsequently screening the viral surface envelope glycoproteins-specific antibodies for their ability to inhibit virus uptake by target cells.

Compositions and immunogenic preparations, including vaccine compositions, comprising the VLPs of the present invention and capable of inducing protective immunity in a suitably treated host and a suitable carrier therefor are provided. "Immunogenic compositions" are those which result in specific antibody production or in cellular immunity when injected into a host. Such immunogenic compositions or vaccines are useful, for example, in immunizing hosts against infection and/or damage caused by viruses, including, but not limited to, HIV, human T-cell leukemia virus (HTLV) type I, SIV, FIV, bovine immunodeficiency virus, bovine leukemia virus and equine infectious anemia virus, SARS, RVFV, Filovirus, Flavivirus, Arena virus, and Bunya virus.

The vaccine preparations can include an immunogenic amount of one or more VLPs, fragment(s), or subunit(s) thereof. Such vaccines can include one or more viral surface envelope glycoproteins and portions thereof, and adjuvant molecule and portions thereof on the surfaces of the VLPs, or in combination with another protein or other immunogen, such as one or more additional virus components naturally associated with viral particles or an epitopic peptide derived therefrom.

By "immunogenic amount" is meant an amount capable of eliciting the production of antibodies directed against the virus, in the host to which the vaccine has been administered. It is preferred for SIV, HIV and HTLV, among others, that the route of administration and the immunogenic composition is designed to optimize the immune response on mucosal surfaces, for example, using nasal administration (via an aerosol) of the immunogenic composition.

Immunogenic carriers can be used to enhance the immunogenicity of the VLPs from any of the viruses discussed herein. Such carriers include, but are not limited to, proteins and polysaccharides, microspheres formulated using (*e.g*., a biodegradable polymer such as DL-lactide-coglycolide, liposomes, and bacterial cells and membranes). Protein carriers may be joined to the proteinases or peptides derived therefrom to form fusion proteins by recombinant or synthetic means or by chemical coupling. Useful carriers and means of coupling such carriers to polypeptide antigens are known in the art.

The immunogenic compositions and/or vaccines may be formulated by any of the means known in the art. They can be typically prepared as injectables or as formulations for intranasal administration, either as liquid solutions or suspensions. Solid forms suitable for solution in, or suspension in, liquid prior to injection or other administration may also be prepared. The preparation may also, for example, be emulsified, or the protein(s)/peptide(s) encapsulated in liposomes.

The active immunogenic ingredients are often mixed with excipients or carriers, which are pharmaceutically acceptable and compatible with the active ingredient. Suitable excipients include but are not limited to water, saline, dextrose, glycerol, ethanol, or the like and combinations thereof. The concentration of the immunogenic polypeptide in injectable, aerosol or nasal formulations is usually in the range of 0.2 to 5 mg/ml. Similar dosages can be administered to other mucosal surfaces.

In addition, if desired, the vaccines may contain minor amounts of auxiliary substances such as wetting or emulsifying agents, pH buffering agents, and/or other agents, which enhance the effectiveness of the vaccine. Examples of agents which may be effective include, but are not limited to: aluminum hydroxide; N-acetyl-muramyl-L-threonyl-D-isoglutamine (thr-MDP); N-acetyl-nor-muramyl-L-alanyl-D-isoglutamine (CGP 11637, referred to as nor-MDP); N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE); and RIBI, which contains three components extracted from bacteria: monophosphoryl lipid A, trehalose dimycolate and cell wall skeleton (MPL+TDM+CWS) in a 2% squalene/Tween 80 emulsion. The effectiveness of the auxiliary substances may be determined by measuring the amount of antibodies (especially IgG, IgM or IgA) directed against the immunogen resulting from administration of the immunogen in vaccines which comprise the adjuvant in question. Additional formulations and modes of administration may also be used.

"Pharmaceutically acceptable salts" include, but are not limited to, the acid addition salts (formed with free amino groups of the peptide) which are formed with inorganic acids (*e.g*., hydrochloric acid or phosphoric acids) and organic acids (*e.g*., acetic, oxalic, tartaric, or maleic acid). Salts formed with the free carboxyl groups may also be derived from inorganic bases (*e.g*., sodium, potassium, ammonium, calcium, or ferric hydroxides), and organic bases (*e.g*., isopropylamine, trimethylamine, 2-ethylamino-ethanol, histidine, and procaine).

The immunogenic compositions and/or vaccines can be administered in a manner compatible with the dosage formulation, and in such amount and manner as will be prophylactically and/or therapeutically effective, according to what is known to the art. The quantity to be administered, which is generally in the range of about 1 to 1,000 micrograms of viral surface envelope glycoprotein per dose and/or adjuvant molecule per dose, more generally in the range of about 5 to 500 micrograms of glycoprotein per dose and/or adjuvant molecule per dose, depends on the subject to be treated, the capacity of the hosts immune system to synthesize antibodies, and the degree of protection desired. Precise amounts of the active ingredient required to be administered may depend on the judgment of the physician or veterinarian and may be peculiar to each individual, but such a determination is within the skill of such a practitioner.

The vaccine or immunogenic composition may be given in a single dose; two dose schedule, for example two to eight weeks apart; or a multiple dose schedule. A multiple dose schedule is one in which a primary course of vaccination may include 1 to 10 or more separate doses, followed by other doses administered at subsequent time intervals as required to maintain and/or reinforce the immune response (*e*.*g*., at 1 to 4 months for a second dose, and if needed, a subsequent dose(s) after several months). Humans (or other animals) immunized with the VLPs of the present invention are protected from infection by the cognate virus.

It should also be noted that the vaccine or immunogenic composition can be used to boost the immunization of a host having been previously treated with a vaccine such as, but not limited to, DNA vaccine and a recombinant virus vaccine.

Except as noted hereafter, standard techniques for peptide synthesis, cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al. (1989) Molecular Cloning, Second Edition, Cold Spring Harbor Laboratory, Plainview, N.Y.; Maniatis et al. (1982) Molecular Cloning, Cold Spring Harbor Laboratory, Plainview, N.Y.; Wu (ed.) (1993) Meth. Enzymol. 218, Part I; Wu (ed.) (1979) Meth. Enzymol. 68; Wu et al. (eds.) (1983) Meth. Enzymol. 100 and 101; Grossman and Moldave (eds.) Meth. Enzymol. 65; Miller (ed.) (1972) Experiments in Molecular Genetics, Cold Spring Harbor Laboratory, Cold Spring Harbor, N.Y., Old Primrose (1981) Principles of Gene Manipulation, University of California Press, Berkeley; Schleif and Wensink (1982) Practical Methods in Molecular Biology; Glover (ed.) (1985) DNA Cloning Vol. I and II, IRL Press, Oxford, UK; Hames and Higgins (eds.) (1985) Nucleic Acid Hybridization, IRL Press, Oxford, UK; Setlow and Hollaender (1979) Genetic Engineering: Principles and Methods, Vols. 1-4, Plenum Press, N.Y.

Abbreviations and nomenclature, where employed, are deemed standard in the field and commonly used in professional journals such as those cited herein.

## Claims

1. A virus-like particle (VLP), comprising:
a viral core protein that can self assemble into a VLP core;
at least one viral surface envelope glycoprotein expressed on the surface of the VLP; and
at least one adjuvant molecule expressed on the surface of the VLP,
wherein at least one adjuvant molecule is from a cell or virus different from the virus from which the viral surface envelope glycoprotein is obtained.

2. The VLP of claim 1, wherein the viral core protein and the viral surface envelope glycoprotein are from different viruses.

3. The VLP of claim 1, wherein the viral core protein and the viral surface envelope glycoprotein are from the same virus.

4. The VLP of claim 1, wherein the viral core protein is selected from an HIV Gag viral core protein, an SIV Gag viral core protein, an MuLV Gag viral core protein, a VSV M viral core protein, an Ebola VP40 viral core protein, a corona virus M protein, a corona virus E protein, a Bunya Virus N Protein viral core protein, and combinations thereof.

5. The VLP of claim 1, wherein the viral surface envelope glycoprotein is selected from a human immunodeficiency virus (HIV), a simian-human immunodeficiency virus (SHIV), a feline immunodeficiency virus (FIV), a feline leukemia virus, a bovine immunodeficiency virus, a bovine leukemia virus, a equine infectious anemia virus, a human T-cell leukemia virus, a Bunya Virus glycoprotein, a Lassa fever virus glycoprotein, an Ebola virus glycoprotein, corona virus glycoprotein, an Arena virus glycoprotein, a Filo virus glycoprotein, an influenza virus glycoprotein, a paramyxovirus glycoprotein, a rhabdo virus glycoprotein, an alphavirus glycoprotein, a flavi virus glycoprotein, and combinations thereof.

6. The VLP of claim 1, wherein the at least one adjuvant molecule is selected from an influenza HA adjuvant molecule, a parainfluenza HN adjuvant molecule, a Venezuelan equine encephalitis (VEE) adjuvant molecule, a Flt3 adjuvant molecule, a C3d adjuvant molecule, a mannose receptor adjuvant molecule, a CD40 adjuvant molecule, and combinations thereof.

7. An immunogenic composition, comprising the VLP of claim 1 and a pharmacologically acceptable carrier.

8. Use of the immunogenic composition of claim 7 for the preparation of a medicament for generating an immunological response in a host.

9. Use of the immunogenic composition of claim 7 for the preparation of a medicament for treating a condition in a host.

10. An in-vitro method of determining exposure of a host to a virus, said method comprising the steps of:
contacting a biological fluid of the host with the virus-like particle (VLP) of claim 1, wherein the VLP is of the same virus type to which exposure is being determined, under conditions which are permissive for binding of antibodies in the biological fluid with the VLP; and
detecting binding of antibodies within the biological fluid with the VLP, whereby exposure of the host to the virus is determined by the detection of antibodies bound to the VLP.

11. The method of claim 10, wherein detecting includes the use of a labeled second antibody which is specific to antibodies in the biological fluid being tested.

12. Use of the immunogenic composition of claim 7 for the preparation of a medicament for targeting antigen presenting cells in a host, wherein the at least one adjuvant molecule is selected from a Venezuelan equine encephalitis (VEE) adjuvant molecule, a Flt3 adjuvant molecule, a C3d adjuvant molecule, a mannose receptor adjuvant molecule, a CD40 adjuvant molecule, and combinations thereof.

13. The use of claim 12, wherein the antigen presenting cell is a dendritic cell and the at least one adjuvant molecule is selected from a Venezuelan equine encephalitis (VEE) adjuvant molecule, a Flt3 adjuvant molecule, a mannose receptor adjuvant molecule, a CD40 adjuvant molecule, and combinations thereof.

14. The use of claim 12, wherein the antigen presenting cell is a follicular dendritic cell and the at least one adjuvant molecule is a C3d adjuvant molecule.

15. Use of the immunogenic composition of claim 7 for the preparation of a medicament for targeting mucosal surfaces in a host, wherein the at least one adjuvant molecule is selected from an influenza HA adjuvant molecule, a parainfluenza HN adjuvant molecule, and combinations thereof.

16. Use of the immunogenic composition of claim 7 for the preparation of a medicament for producing humoral and cellular immune responses in mucosal and systemic compartments, wherein the at least one viral surface envelope glycoprotein is selected from human immunodeficiency virus (HIV), and wherein the at least one adjuvant molecule is selected from an influenza HA adjuvant molecule, a parainfluenza HN adjuvant molecule, and combinations thereof.

17. A method of making a virus-like particle (VLP), comprising the steps of:
providing a viral core protein expression vector, wherein the viral core protein is capable of self-assembling into a VLP core;
providing a viral surface envelope glycoprotein expression vector;
providing a adjuvant molecule expression vector; and
introducing into a cell the viral core protein expression vector, the viral surface envelope glycoprotein expression vector, and the adjuvant molecule expression vector and allowing for expression of the viral surface envelope glycoprotein and the adjuvant molecule,
whereby the VLP is formed by the cells.

18. The method of claim 17, wherein the VLP is selected from human immunodeficiency virus (HIV) VLP, a simian-human immunodeficiency virus (SHIV) VLP, a feline immunodeficiency virus (FIV) VLP, a feline leukemia virus VLP, a bovine immunodeficiency virus VLP, a bovine leukemia virus VLP, a equine infectious anemia virus VLP, a human T-cell leukemia virus VLP, a Bunya Virus VLP, Lassa fever virus VLP, Ebola virus VLP, corona virus VLP, Arena virus VLP, Filovirus VLP, influenza virus VLP, paramyxovirus VLP, rhabdo virus VLP, alphavirus VLP, and flavi virus VLP.

19. A method of making a virus-like particle (VLP), comprising the steps of:
operably linking a coding sequence for a viral core protein of a virus to a promoter, and inserting it into a viral core protein expression vector, wherein the viral core protein is capable of self-assembling into a VLP core;
operably linking a coding sequence for a viral surface envelope glycoprotein of a virus to a promoter, and inserting it into a viral surface envelope glycoprotein expression vector;
operably linking a coding sequence for an adjuvant molecule to a promoter, and inserting it into a adjuvant molecule expression vector;
simultaneously introducing into a cell the viral core protein expression vector, the viral surface envelope glycoprotein expression vector, and the adjuvant molecule expression vector and allowing for expression of the viral surface envelope glycoprotein and the adjuvant molecule,
whereby the VLP is formed by the cells.

20. The method of claim 19, wherein the promoter is selected from a baculovirus promoter and a recombinant modified vaccinia Ankara (MVA) promoter, a CMV promoter, EF promoter, adenovirus promoter, recombinant VSV, recombinant adenovirus, recombinant alphavirus, and recombinant DNA expression vectors.

21. Use of the immunogenic composition of claim 7 for the preparation of a medicament for boosting the immunization of a host having been previously treated with a vaccine.

## Patentansprüche

1. Virus-ähnliches Partikel (VLP), umfassend:
ein virales Kernprotein, das selbst in einen VLP-Kern assemblieren kann;
mindestens ein virales Oberflächen-Hüll-Glycoprotein, das auf der Oberfläche des VLP exprimiert wird; und
mindestens ein Hilfsmolekül, das auf der Oberfläche des VLP exprimiert wird,
wobei mindestens ein Hilfsmolekül aus einer Zelle oder einem Virus stammt, die/der unterschiedlich zum Virus ist, aus dem das virale Oberflächen-Hüll-Glycoprotein erhalten wird.

2. VLP nach Anspruch 1, wobei das virale Kernprotein und das virale Oberflächen-Hüll-Glycoprotein aus unterschiedlichen Viren stammen.

3. VLP nach Anspruch 1, wobei das virale Kernprotein und das virale Oberflächen-Hüll-Glycoprotein aus dem gleichen Virus stammen.

4. VLP nach Anspruch 1, wobei das virale Kernprotein ausgewählt ist aus einem viralen Kernprotein HIV-Gag, einem viralen Kernprotein SIV-Gag, einem viralen Kernprotein MuLV Gag, einem viralen Kernprotein VSV M, einem viralen Kernprotein Ebola VP40, einem Coronavirus M-Protein, einem Coronavirus E-Protein, einem viralen Kernprotein Bunyavirus N-Protein und Kombinationen davon.

5. VLP nach Anspruch 1, wobei das virale Oberflächen-Hüll-Glycoprotein ausgewählt ist aus einem Humanen Immundefizienz Virus (HIV), einem Simian-Humanen Immundefizienz Virus (SHIV), einem Felinen Immundefizienz Virus (FIV), einem Felinen Leukämie Virus, einem Bovinen Immundefizienz Virus, einem Bovinen Leukämie Virus, einem Equinen Infektiösen Anämievirus, einem Humanen T-Zell Leukämie Virus, einem Bunyavirus Glycoprotein, einem Lassafiebervirus Glycoprotein, einem Ebolavirus Glycoprotein, einem Coronavirus Glycoprotein, einem Arenavirus Glycoprotein, einem Filovirus Glycoprotein, einem Influenzavirus Glycoprotein, einem Paramyxovirus Glycoprotein, einem Rhabdovirus Glycoprotein, einem Alphavirus Glycoprotein, einem Flavivirus Glycoprotein und Kombinationen davon.

6. VLP nach Anspruch 1, wobei das mindestens eine Hilfsmolekül ausgewählt ist aus einem Influenza HA-Hilfsmolekül, einem Parainfluenza HN-Hilfsmolekül, einem Hilfsmolekül der Venezolanischen Pferdeenzephalomyelitis, einem Flt3-Hilfsmolekül, einem C3d-Hilfsmolekül, einem Mannose-Rezeptor-Hilfsmolekül, einem CD40-Hilfsmolekül und Kombinationen davon.

7. Immunogene Zusammensetzung, umfassend VLP nach Anspruch 1 und einen pharmakologisch akzeptablen Träger.

8. Verwendung einer immunogenen Zusammensetzung nach Anspruch 7 zur Herstellung eines Medikaments zur Erzeugung einer immunologischen Antwort in einem Wirt.

9. Verwendung einer immunogenen Zusammensetzung nach Anspruch 7 zur Herstellung eines Medikaments zur Behandlung eines Zustands in einem Wirt.

10. in vitro-Verfahren zur Bestimmung einer Aussetzung eines Wirts gegenüber einem Virus, wobei das Verfahren die Schritte umfasst:
Kontaktieren einer biologischen Flüssigkeit des Wirts mit dem Virus-ähnlichen Partikel (VLP) nach Anspruch 1, wobei das VLP aus dem gleichen Virus stammt, gegenüber welchem eine Aussetzung bestimmt wird, unter Bedingungen, die eine Bindung von Antikörpern in der biologischen Flüssigkeit mit dem VLP zulassen; und
Detektieren der Bindung von Antikörpern innerhalb der biologischen Flüssigkeit mit dem VLP, wobei eine Aussetzung des Wirts gegenüber dem Virus durch die Detektion von an das VLP gebundenen Antikörpern bestimmt wird.

11. Verfahren nach Anspruch 10, wobei Detektieren die Verwendung eines markierten zweiten Antikörpers einschließt, der spezifisch für Antikörper in der biologischen Flüssigkeit, die getestet wird, ist.

12. Verwendung der immunogenen Zusammensetzung nach Anspruch 7 zur Herstellung eines Medikaments zum Anvisieren von Antigenpräsentierenden Zellen in einem Wirt, wobei das mindestens eine Hilfsmolekül ausgewählt ist aus einem Hilfsmolekül der Venezolanischen Pferdeenzephalomyelitis (VEE), einem Flt3-Hilfsmolekül, einem C3d-Hilfsmolekül, einem Mannose-Rezeptor-Hilfsmolekül, einem CD40-Hilfsmolekül und Kombinationen davon.

13. Verwendung nach Anspruch 12, wobei die Antigen-präsentierende Zelle eine dendritische Zelle ist und das mindestens eine Hilfsmolekül ausgewählt ist aus einem Hilfsmolekül der Venezolanischen Pferdeenzephalomyelitis (VEE), einem Flt3-Hilfsmolekül, einem Mannose-Rezeptor-Hilfsmolekül, einem CD40-Hilfsmolekül und Kombinationen davon.

14. Verwendung nach Anspruch 12, wobei die Antigen-präsentierende Zelle eine follikuläre dendritische Zelle ist und das mindestens eine Hilfsmolekül ein C3d-Hilfsmolekül ist.

15. Verwendung der immunogenen Zusammensetzung nach Anspruch 7 zur Herstellung eines Medikaments zum Anvisieren von Mucosa-Oberflächen in einem Wirt, wobei das mindestens eine Hilfsmolekül ausgewählt ist aus einem Influenza HA-Hilfsmolekül, einem Parainfluenza HN-Hilfsmolekül und Kombinationen davon.

16. Verwendung der immunogenen Zusammensetzung nach Anspruch 7 zur Herstellung eines Medikaments zur Herstellung humoraler und zellulärer Immunantworten in Mucosa- und systemischen Kompartimenten, wobei das mindestens eine virale Oberflächen-Hüll- Glycoprotein ausgewählt ist aus Humanem Immundefizienz Virus (HIV), und wobei das mindestens eine Hilfsmolekül ausgewählt ist aus einem Influenza HA-Hilfsmolekül, einem Parainfluenza HN-Hilfsmolekül und Kombinationen davon.

17. Verfahren zur Herstellung eines Virus-ähnlichen Partikels (VLP), umfassend die Schritte:
Bereitstellen eines Expressionsvektors für virales Kernprotein, wobei das virale Kernprotein fähig ist, in einem VLP-Kern selbst zu assemblieren;
Bereitstellen eines Expressionsvektors für virales Oberflächen-Hüll-Glycoprotein; Bereitstellen eines Expressionsvektors für Hilfsmolekül; und
Einführen des Expressionsvektors für virales Kernprotein, des Expressionsvektors für virales Oberflächen-Hüll-Glycoprotein und des Expressionsvektors für Hilfsmolekül in eine Zelle, und Erlauben der Expression des viralen Oberflächen-Hüll-Glycoproteins und des Hilfsmoleküls, wodurch das VLP durch die Zellen gebildet wird.

18. Verfahren nach Anspruch 17, wobei das VLP ausgewählt ist aus Humanem Immundefizienz Virus (HIV) VLP, einem Simian-Humanen Immundefizienz Virus (SHIV) VLP, einem Felinen Immundefizienz Virus (FIV) VLP, einem Felinen Leukämie Virus VLP, einem Bovinen Immundefizienz Virus VLP, einem Bovinen Leukämie Virus VLP, einem Equinen Infektiösen Anämievirus VLP, einem Humanen T-Zell Leukämie Virus VLP, einem Bunyavirus VLP, einem Lassafiebervirus VLP, einem Ebolavirus VLP, einem Coronavirus VLP, einem Arenavirus VLP, einem Filovirus VLP, einem Influenzavirus VLP, einem Paramyxovirus VLP, einem Rhabdovirus VLP, einem Alphavirus VLP und einem Flavivirus VLP.

19. Verfahren zur Herstellung eines Virus-ähnlichen Partikels (VLP), umfassend die Schritte:
operatives Verknüpfen einer Kodierungssequenz für ein virales Kernprotein eines Virus an einen Promotor und Einfügen dieser in einen Expressionsvektor für virales Kernprotein; wobei das virale Kernprotein fähig ist, in einem VLP-Kern selbst zu assemblieren;
operatives Verknüpfen einer Kodierungssequenz für ein virales Oberflächen-Hüll-Glycoprotein eines Virus an einen Promotor, und Einfügen dieser in einen Expressionsvektor für virales Oberflächen-Hüll-Glycoprotein;
operatives Verknüpfen einer Kodierungssequenz für ein Hilfsmolekül an einen Promotor, und Einfügen dieser in einen Expressionsvektor für Hilfsmolekül;
gleichzeitig Einführen des Expressionsvektors für virales Kernprotein, des Expressionsvektors für virales Oberflächen-Hüll-Glycoprotein und des Expressionsvektors für Hilfsmolekül in eine Zelle, und Erlauben der Expression des viralen Oberflächen-Hüll-Glycoproteins und des Hilfsmoleküls, wodurch das VLP durch die Zellen gebildet wird.

20. Verfahren nach Anspruch 19, wobei der Promoter ausgewählt ist aus einem Baculovirus Promoter und einem rekombinanten Modifiziertem Vaccinia Ankara (MVA) Promoter, einem CMV Promoter, EF Promoter, Adenovirus Promoter, rekombinanten VSV, rekombinanten Adenovirus, rekombinanten Alphavirus und rekombinanten DNA-Expressionsvektoren.

21. Verwendung der immunogenen Zusammensetzung nach Anspruch 7 zur Herstellung eines Medikaments zur Verstärkung der Immunisierung eines Wirts, der vorher mit einem Vakzin behandelt wurde.

## Revendications

1. Particule pseudovirale (VLP), comprenant :
une protéine virale centrale qui peut s'autoassembler à l'intérieur d'un noyau de VLP ;
au moins une glycoprotéine d'enveloppe de surface de virus exprimée à la surface de la VLP ; et
au moins une molécule d'adjuvant exprimée à la surface de la VLP,
dans laquelle au moins une molécule d'adjuvant provient d'une cellule ou d'un virus différent(e) du virus à partir duquel est obtenue la glycoprotéine d'enveloppe de surface de virus.

2. VLP selon la revendication 1, dans laquelle la protéine virale centrale et la glycoprotéine d'enveloppe de surface de virus proviennent de virus différents.

3. VLP selon la revendication 1, dans laquelle la protéine virale centrale et la glycoprotéine d'enveloppe de surface de virus proviennent du même virus.

4. VLP selon la revendication 1, dans laquelle la protéine virale centrale est choisie parmi une protéine virale centrale Gag du virus de l'immunodéficience humaine (VIH), une protéine virale centrale Gag du virus de l'immunodéficience simienne (SIV), une protéine virale centrale Gag du virus de la leucémie murine (MuLV), une protéine virale centrale M du virus de la stomatite vésiculaire (VSV), une protéine virale centrale VP40 du virus Ebola, une protéine M du coronavirus, une protéine E du coronavirus, une protéine virale centrale de la protéine N du Bunyavirus, et des combinaisons de celles-ci.

5. VLP selon la revendication 1, dans laquelle la glycoprotéine d'enveloppe de surface de virus est choisie parmi une glycoprotéine du virus de l'immunodéficience humaine (VIH), une glycoprotéine du virus de l'immunodéficience humaine-simienne (SHIV), une glycoprotéine du virus de l'immunodéficience féline (FIV), une glycoprotéine du virus de la leucémie féline, une glycoprotéine du virus de l'immunodéficience bovine, une glycoprotéine du virus de la leucémie bovine, une glycoprotéine du virus de l'anémie infectieuse équine, une glycoprotéine du virus de la leucémie humaine à lymphocytes T, une glycoprotéine du Bunyavirus, une glycoprotéine du virus de la fièvre de Lassa, une glycoprotéine du virus Ebola, une glycoprotéine du coronavirus, une glycoprotéine de l'Arénavirus, une glycoprotéine du Filovirus, une glycoprotéine du virus de la grippe, une glycoprotéine du paramyxovirus, une glycoprotéine du rhabdovirus, une glycoprotéine de l'alphavirus, une glycoprotéine du flavivirus, et des combinaisons de celles-ci.

6. VLP selon la revendication 1, dans laquelle l'au moins une molécule d'adjuvant est choisie parmi une molécule d'adjuvant HA du virus de la grippe, une molécule d'adjuvant HN du virus paragrippal, une molécule d'adjuvant du virus de l'encéphalite équine vénézuélienne (EEV), une molécule d'adjuvant du Flt3, une molécule d'adjuvant du C3d, une molécule d'adjuvant du récepteur de mannose, une molécule d'adjuvant du CD40, et des combinaisons de celles-ci.

7. Composition immunogène, comprenant la VLP selon la revendication 1 et un transporteur pharmacologiquement acceptable.

8. Utilisation de la composition immunogène selon la revendication 7 pour la préparation d'un médicament destiné à générer une réponse immunologique chez un hôte.

9. Utilisation de la composition immunogène selon la revendication 7 pour la préparation d'un médicament destiné à traiter un état pathologique chez un hôte.

10. Procédé *in vitro* destiné à déterminer l'exposition d'un hôte à un virus, ledit procédé comprenant les étapes consistant à :
mettre en contact un fluide biologique de l'hôte avec la particule pseudovirale (VLP) selon la revendication 1, la VLP provenant du même type de virus que celui pour lequel l'exposition doit être déterminée, dans des conditions permettant la liaison des anticorps dans le fluide biologique avec la VLP ; et
détecter la liaison des anticorps à l'intérieur du fluide biologique avec la VLP, l'exposition de l'hôte au virus étant déterminée par la détection des anticorps liés à la VLP.

11. Procédé selon la revendication 10, dans lequel l'étape de détection comprend l'utilisation d'un second anticorps marqué qui est spécifique des anticorps présents dans le fluide biologique testé.

12. Utilisation de la composition immunogène selon la revendication 7 pour la préparation d'un médicament destiné à cibler des cellules présentatrices d'antigènes chez un hôte, dans laquelle l'au moins une molécule d'adjuvant est choisie parmi une molécule d'adjuvant du virus de l'encéphalite équine vénézuélienne (EEV), une molécule d'adjuvant du Flt3, une molécule d'adjuvant du C3d, une molécule d'adjuvant du récepteur du mannose, une molécule d'adjuvant du CD40, et des combinaisons de celles-ci.

13. Utilisation selon la revendication 12, dans laquelle la cellule présentatrice d'antigènes est une cellule dendritique et l'au moins une molécule d'adjuvant est choisie parmi une molécule d'adjuvant du virus de l'encéphalite équine vénézuélienne (EEV), une molécule d'adjuvant du Flt3, une molécule d'adjuvant du récepteur du mannose, une molécule d'adjuvant du CD40, et des combinaisons de celles-ci.

14. Utilisation selon la revendication 12, dans laquelle la cellule présentatrice d'antigènes est une cellule dendritique folliculaire et l'au moins une molécule d'adjuvant est une molécule d'adjuvant du C3d.

15. Utilisation de la composition immunogène selon la revendication 7 pour la préparation d'un médicament destiné à cibler les surfaces des muqueuses chez un hôte, dans laquelle l'au moins une molécule d'adjuvant est choisie parmi une molécule d'adjuvant HA du virus de la grippe, une molécule d'adjuvant HN du virus paragrippal, et des combinaisons de celles-ci.

16. Utilisation de la composition immunogène selon la revendication 7 pour la préparation d'un médicament destiné à produire des réponses immunitaires humorales et cellulaires dans les compartiments systémiques et ceux des muqueuses, dans laquelle l'au moins une glycoprotéine d'enveloppe de surface de virus est choisie parmi une glycoprotéine du virus de l'immunodéficience humaine (VIH), et dans laquelle l'au moins une molécule d'adjuvant est choisie parmi une molécule d'adjuvant HA du virus de la grippe, une molécule d'adjuvant HN du virus paragrippal, et des combinaisons de celles-ci.

17. Procédé de fabrication d'une particule pseudovirale (VLP), comprenant les étapes suivantes :
fournir un vecteur d'expression de la protéine virale centrale, la protéine virale centrale étant capable de s'auto-assembler à l'intérieur d'un noyau de VLP ;
fournir un vecteur d'expression de la glycoprotéine d'enveloppe de surface du virus ;
fournir un vecteur d'expression de la molécule d'adjuvant ; et
introduire le vecteur d'expression de la protéine virale centrale, le vecteur d'expression de la glycoprotéine d'enveloppe de surface du virus, et le vecteur d'expression de la molécule d'adjuvant à l'intérieur d'une cellule et permettre l'expression de la glycoprotéine d'enveloppe de surface du virus et de la molécule d'adjuvant,
la VLP étant ainsi formée par les cellules.

18. Procédé selon la revendication 17, dans lequel la VLP est choisie parmi une VLP du virus de l'immunodéficience humaine (VIH), une VLP du virus de l'immunodéficience humaine-simienne (SHIV), une VLP du virus de l'immunodéficience féline (FIV), une VLP du virus de la leucémie féline, une VLP du virus de l'immunodéficience bovine, une VLP du virus de la leucémie bovine, une VLP du virus de l'anémie infectieuse équine, une VLP du virus de la leucémie humaine à lymphocytes T, une VLP du Bunyavirus, une VLP du virus de la fièvre de Lassa, une VLP du virus Ebola, une VLP du coronavirus, une VLP de l'Arénavirus, une VLP du Filovirus, une VLP du virus de la grippe, une VLP du paramyxovirus, une VLP du rhabdovirus, une VLP de l'alphavirus, et une VLP du flavivirus.

19. Procédé de fabrication d'une particule pseudovirale (VLP), comprenant les étapes suivantes :
lier de manière fonctionnelle une séquence codant pour une protéine virale centrale d'un virus à un promoteur, et l'insérer à l'intérieur d'un vecteur d'expression d'une protéine centrale virale, la protéine centrale virale étant capable de s'auto-assembler à l'intérieur d'un noyau de VLP ;
lier de manière fonctionnelle une séquence codant pour une glycoprotéine d'enveloppe de surface du virus d'un virus à un promoteur, et l'insérer à l'intérieur d'un vecteur d'expression d'une glycoprotéine d'enveloppe de surface de virus ;
lier de manière fonctionnelle une séquence codant pour une molécule d'adjuvant à un promoteur, et l'insérer à l'intérieur d'un vecteur d'expression d'une molécule d'adjuvant ;
introduire simultanément le vecteur d'expression de la protéine virale centrale, le vecteur d'expression de la glycoprotéine d'enveloppe de surface de virus, et le vecteur d'expression de la molécule d'adjuvant à l'intérieur d'une cellule et permettre l'expression de la glycoprotéine d'enveloppe de surface de virus et de la molécule d'adjuvant,
la VLP étant ainsi formée par les cellules.

20. Procédé selon la revendication 19, dans lequel le promoteur est choisi parmi un promoteur de baculovirus et un promoteur du virus de la vaccine Ankara modifié (MVA) recombinant, un promoteur du CMV, un promoteur du EF, un promoteur de l'adénovirus, du VSV recombinant, de l'adénovirus recombinant, de l'alphavirus recombinant, et des vecteurs d'expression d'ADN recombinant.

21. Utilisation de la composition immunogène selon la revendication 7 pour la préparation d'un médicament destiné à renforcer l'immunisation d'un hôte ayant été précédemment traité avec un vaccin.


### SEQUENCE LISTING

<110> Emory University
<120> Virus-Like Particles, Methods of Preparation, And Immunogenic Compositions
<130> 050508-2210
<140> TBA
   <141> 2003-05-19
<150> 60/381,557
   <151> 2002-05-17
<160> 11
<170> PatentIn version 3.2
<210> 1
   <211> 847
   <212> PRT
   <213> HIV SF162 Envelope Protein
<400> 1
   Met Arg Val Lys Gly Ile Arg Lys Asn Tyr Gln His Leu Trp Arg Gly
   1 5 10 15
   Gly Thr Leu Leu Leu Gly Met Leu Met Ile Cys Ser Ala Val Glu Lys
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Gln Glu Ile Val Leu Glu Asn Val Thr Glu Asn Phe Asn Met Trp Lys
   85 90 95
   Asn Asn Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   His Cys Thr Asn Leu Lys Asn Ala Thr Asn Thr Lys Ser Ser Asn Trp
   130 135 140
   Lys Glu Met Asp Arg Gly Glu Ile Lys Asn Cys Ser Phe Lys Val Thr
   145 150 155 160
   Thr Ser Ile Arg Asn Lys Met Gln Lys Glu Tyr Ala Leu Phe Tyr Lys
   165 170 175
   Leu Asp Val Val Pro Ile Asp Asn Asp Asn Thr Ser Tyr Lys Leu Ile
   180 185 190
   Asn Cys Asn Thr Ser Val Ile Thr Gln Ala Cys Pro Lys Val Ser Phe
   195 200 205
   Glu Pro Ile Pro Ile His Tyr Cys Ala Pro Ala Gly Phe Ala Ile Leu
   210 215 220
   Lys Cys Asn Asp Lys Lys Phe Asn Gly Ser Gly Pro Cys Thr Asn Val
   225 230 235 240
   Ser Thr Val Gln Cys Thr His Gly Ile Arg Pro Val Val Ser Thr Gln
   245 250 255
   Leu Leu Leu Asn Gly Ser Leu Ala Glu Glu Gly Val Val Ile Arg Ser
   260 265 270
   Glu Asn Phe Thr Asp Asn Ala Lys Thr Ile Ile Val Gln Leu Lys Glu
   275 280 285
   Ser Val Glu Ile Asn Cys Thr Arg Pro Asn Asn Asn Thr Arg Lys Ser
   290 295 300
   Ile Thr Ile Gly Pro Gly Arg Ala Phe Tyr Ala Thr Gly Asp Ile Ile
   305 310 315 320
   Gly Asp Ile Arg Gln Ala His Cys Asn Ile Ser Gly Glu Lys Trp Asn
   325 330 335
   Asn Thr Leu Lys Gln Ile Val Thr Lys Leu Gln Ala Gln Phe Gly Asn
   340 345 350
   Lys Thr Ile Val Phe Lys Gln Ser Ser Gly Gly Asp Pro Glu Ile Val
   355 360 365
   Met His Ser Phe Asn Cys Gly Gly Glu Phe Phe Tyr Cys Asn Ser Thr
   370 375 380
   Gln Leu Phe Asn Ser Thr Trp Asn Asn Thr Ile Gly Pro Asn Asn Thr
   385 390 395 400
   Asn Gly Thr Ile Thr Leu Pro Cys Arg Ile Lys Gln Ile Ile Asn Arg
   405 410 415
   Trp Gln Glu Val Gly Lys Ala Met Tyr Ala Pro Pro Ile Arg Gly Gln
   420 425 430
   Ile Arg Cys Ser Ser Asn Ile Thr Gly Leu Leu Leu Thr Arg Asp Gly
   435 440 445
   Gly Lys Glu Ile Ser Asn Thr Thr Glu Ile Phe Arg Pro Gly Gly Gly
   450 455 460
   Asp Met Arg Asp Asn Trp Arg Ser Glu Leu Tyr Lys Tyr Lys Val Val
   465 470 475 480
   Lys Ile Glu Pro Leu Gly Val Ala Pro Thr Lys Ala Lys Arg Arg Val
   485 490 495
   Val Gln Arg Glu Lys Arg Ala Val Thr Leu Gly Ala Met Phe Leu Gly
   500 505 510
   Phe Leu Gly Ala Ala Gly Ser Thr Met Gly Ala Arg Ser Leu Thr Leu
   515 520 525
   Thr Val Gln Ala Arg Gln Leu Leu Ser Gly Ile Val Gln Gln Gln Asn
   530 535 540
   Asn Leu Leu Arg Ala Ile Glu Ala Gln Gln His Leu Leu Gln Leu Thr
   545 550 555 560
   Val Trp Gly Ile Lys Gln Leu Gln Ala Arg Val Leu Ala Val Glu Arg
   565 570 575
   Tyr Leu Lys Asp Gln Gln Leu Leu Gly Ile Trp Gly Cys Ser Gly Lys
   580 585 590
   Leu Ile Cys Thr Thr Ala Val Pro Trp Asn Ala Ser Trp Ser Asn Lys
   595 600 605
   Ser Leu Asp Gln Ile Trp Asn Asn Met Thr Trp Met Glu Trp Glu Arg
   610 615 620
   Glu Ile Asp Asn Tyr Thr Asn Leu Ile Tyr Thr Leu Ile Glu Glu Ser
   625 630 635 640
   Gln Asn Gln Gln Glu Lys Asn Glu Gln Glu Leu Leu Glu Leu Asp Lys
   645 650 655
   Trp Ala Ser Leu Trp Asn Trp Phe Asp Ile Ser Lys Trp Leu Trp Tyr
               660                 665                 670
   Ile Lys Ile Phe Ile Met Ile Val Gly Gly Leu Val Gly Leu Arg Ile
           675                 680                 685
   Val Phe Thr Val Leu Ser Ile Val Asn Arg Val Arg Gln Gly Tyr Ser
       690                 695                 700
   Pro Leu Ser Phe Gln Thr Arg Phe Pro Ala Pro Arg Gly Pro Asp Arg
   705                 710                 715                 720
   Pro Glu Gly Ile Glu Glu Glu Gly Gly Glu Arg Asp Arg Asp Arg Ser
                   725                 730                 735
   Ser Pro Leu Val His Gly Leu Leu Ala Leu Ile Trp Asp Asp Leu Arg
               740                 745                 750
   Ser Leu Cys Leu Phe Ser Tyr His Arg Leu Arg Asp Leu Ile Leu Ile
           755                 760                 765
   Ala Ala Arg Ile Val Glu Leu Leu Gly Arg Arg Gly Trp Glu Ala Leu
       770                 775                 780
   Lys Tyr Trp Gly Asn Leu Leu Gln Tyr Trp Ile Gln Glu Leu Lys Asn
   785                 790                 795                 800
   Ser Ala Val Ser Leu Phe Asp Ala Ile Ala Ile Ala Val Ala Glu Gly
                   805                 810                 815
   Thr Asp Arg Ile Ile Glu Val Ala Gln Arg Ile Gly Arg Ala Phe Leu
               820                 825                 830
   His Ile Pro Arg Arg Ile Arg Gln Gly Phe Glu Arg Ala Leu Leu
           835                 840                 845
<210> 2
   <211> 1067
   <212> PRT
   <213> RVFV Glycoprotein a.a.
<400> 2
   Met Ala Gly Ile Ala Met Thr Val Leu Pro Ala Leu Ala Val Phe Ala
   1               5                   10                  15
   Leu Ala Pro Val Val Phe Ala Glu Asp Pro His Leu Arg Asn Arg Pro
               20                  25                  30
   Gly Lys Gly His Asn Tyr Ile Asp Gly Met Thr Gln Glu Asp Ala Thr
           35                  40                  45
   Cys Lys Pro Val Thr Tyr Ala Gly Ala Cys Ser Ser Phe Asp Val Leu
       50                  55                  60
   Leu Glu Lys Gly Lys Phe Pro Leu Phe Gln Ser Tyr Ala His His Arg
   65                  70                  75                  80
   Thr Leu Leu Glu Ala Val His Asp Thr Ile Ile Ala Lys Ala Asp Pro
                   85                  90                  95
   Pro Ser Cys Asp Leu Gln Ser Ala His Gly Asn Pro Cys Met Lys Glu
               100                 105                 110
   Lys Leu Val Met Lys Thr His Cys Pro Asn Asp Tyr Gln Ser Ala His
           115                 120                 125
   Tyr Leu Asn Asn Asp Gly Lys Met Ala Ser Val Lys Cys Pro Pro Lys
       130                 135                 140
   Tyr Gly Leu Thr Glu Asp Cys Asn Phe Cys Arg Gln Met Thr Gly Ala
   145                 150                 155                 160
   Ser Leu Lys Lys Gly Ser Tyr Pro Leu Gln Asp Leu Phe Cys Gln Ser
                   165                 170                 175
   Ser Glu Asp Asp Gly Ser Lys Leu Lys Thr Lys Met Lys Gly Val Cys
   180 185 190
   Glu Val Gly Val Gln Ala His Lys Lys Cys Asp Gly Gln Leu Ser Thr
   195 200 205
   Ala His Glu Val Val Pro Phe Ala Val Phe Lys Asn Ser Lys Lys Val
   210 215 220
   Tyr Leu Asp Lys Leu Asp Leu Lys Thr Glu Glu Asn Leu Leu Pro Asp
   225 230 235 240
   Ser Phe Val Cys Phe Glu His Lys Gly Gln Tyr Lys Gly Thr Met Asp
   245 250 255
   Ser Gly Gln Thr Lys Arg Glu Leu Lys Ser Phe Asp Ile Ser Gln Cys
   260 265 270
   Pro Lys Ile Gly Gly His Gly Ser Lys Lys Cys Thr Gly Asp Ala Ala
   275 280 285
   Phe Cys Ser Ala Tyr Glu Cys Thr Ala Gln Tyr Ala Asn Ala Tyr Cys
   290 295 300
   Ser His Ala Asn Gly Ser Gly Ile Val Gln Ile Gln Val Ser Gly Val
   305 310 315 320
   Trp Lys Lys Pro Leu Cys Val Gly Tyr Glu Arg Val Val Val Lys Arg
   325 330 335
   Glu Leu Ser Ala Lys Pro Ile Gln Arg Val Glu Pro Cys Thr Thr Cys
   340 345 350
   Ile Thr Lys Cys Glu Pro His Gly Leu Val Val Arg Ser Thr Gly Phe
   355 360 365
   Lys Ile Ser Ser Ala Val Ala Cys Ala Ser Gly Val Cys Val Thr Gly
       370                 375                 380
   Ser Gln Ser Pro Ser Thr Glu Ile Thr Leu Lys Tyr Pro Gly Ile Ser
   385                 390                 395                 400
   Gln Ser Ser Gly Gly Asp Ile Gly Val His Met Ala His Asp Asp Gln
                   405                 410                 415
   Ser Val Ser Ser Lys Ile Val Ala His Cys Pro Pro Gln Asp Pro Cys
               420                 425                 430
   Leu Val His Gly Cys Ile Val Cys Ala His Gly Leu Ile Asn Tyr Gln
           435                 440                 445
   Cys His Thr Ala Leu Ser Ala Phe Val Val Val Phe Val Phe Ser Ser
       450                 455                 460
   Ile Ala Ile Ile Cys Leu Ala Val Leu Tyr Arg Val Leu Lys Cys Leu
   465                 470                 475                 480
   Lys Ile Ala Pro Arg Lys Val Leu Asn Pro Leu Met Trp Ile Thr Ala
                   485                 490                 495
   Phe Ile Arg Trp Ile Tyr Lys Lys Met Val Ala Arg Val Ala His Asn
               500                 505                 510
   Ile Asn Gln Val Asn Arg Glu Ile Gly Trp Met Glu Gly Gly Gln Leu
           515                 520                 525
   Val Leu Gly Asn Pro Ala Pro Ile Pro Arg His Ala Pro Ile Pro Arg
       530                 535                 540
   Tyr Ser Thr Tyr Leu Met Leu Leu Leu Ile Val Ser Tyr Ala Ser Ala
   545                 550                 555                 560
   Cys Ser Glu Leu Ile Gln Ala Ser Ser Arg Ile Thr Thr Cys Ser Thr
   565 570 575
   Glu Gly Val Asn Thr Lys Cys Arg Leu Ser Gly Thr Ala Leu Ile Arg
   580 585 590
   Ala Gly Ser Val Gly Ala Glu Ala Cys Leu Met Leu Lys Gly Val Lys
   595 600 605
   Glu Asp Gln Thr Lys Phe Leu Lys Ile Lys Thr Val Ser Ser Glu Leu
   610 615 620
   Ser Cys Arg Glu Gly Gln Ser Tyr Trp Thr Gly Ser Ile Ser Pro Lys
   625 630 635 640
   Cys Leu Ser Ser Arg Arg Cys His Leu Val Gly Glu Cys His Val Asn
   645 650 655
   Arg Cys Leu Ser Trp Arg Asp Asn Glu Thr Ser Ala Glu Phe Ser Phe
   660 665 670
   Val Gly Glu Ser Thr Thr Met Arg Glu Asn Lys Cys Phe Glu Gln Cys
   675 680 685
   Gly Gly Trp Gly Cys Gly Cys Phe Asn Val Asn Pro Ser Cys Leu Phe
   690 695 700
   Val His Thr Tyr Leu Gln Ser Val Arg Lys Glu Ala Leu Arg Val Phe
   705 710 715 720
   Asn Cys Ile Asp Trp Val His Lys Leu Thr Leu Glu Ile Thr Asp Phe
   725 730 735
   Asp Gly Ser Val Ser Thr Ile Asp Leu Gly Ala Ser Ser Ser Arg Phe
   740 745 750
   Thr Asn Trp Gly Ser Val Ser Leu Ser Leu Asp Ala Glu Gly Ile Ser
           755                 760                 765
   Gly Ser Asn Ser Phe Ser Phe Ile Glu Ser Pro Ser Lys Gly Tyr Ala
       770                 775                 780
   Ile Val Asp Glu Pro Phe Ser Glu Ile Pro Arg Gln Gly Phe Leu Gly
   785                 790                 795                 800
   Glu Ile Arg Cys Asn Ser Glu Ser Ser Val Leu Ser Ala His Glu Ser
                   805                 810                 815
   Cys Leu Arg Ala Pro Asn Leu Ile Ser Tyr Lys Pro Met Ile Asp Gln
               820                 825                 830
   Leu Glu Cys Thr Thr Asn Leu Ile Asp Pro Phe Val Val Phe Glu Arg
           835                 840                 845
   Gly Ser Leu Pro Gln Thr Arg Asn Asp Lys Thr Phe Ala Ala Ser Lys
       850                 855                 860
   Gly Asn Arg Gly Val Gln Ala Phe Ser Lys Gly Ser Val Gln Ala Asp
   865                 870                 875                 880
   Leu Thr Leu Met Phe Asp Asn Phe Glu Val Asp Phe Val Gly Ala Ala
                   885                 890                 895
   Val Ser Cys Asp Ala Ala Phe Leu Asn Leu Thr Gly Cys Tyr Ser Cys
               900                 905                 910
   Asn Ala Gly Ala Arg Val Cys Leu Ser Ile Thr Ser Thr Gly Thr Gly
           915                 920                 925
   Ser Leu Ser Ala His Asn Lys Asp Gly Ser Leu His Ile Val Leu Pro
       930                 935                 940
   Ser Glu Asn Gly Thr Lys Asp Gln Cys Gln Ile Leu His Phe Thr Val
   945 950 955 960
   Pro Glu Val Glu Glu Glu Phe Met Tyr Ser Cys Asp Gly Asp Glu Arg
   965 970 975
   Pro Leu Leu Val Lys Gly Thr Leu Ile Ala Ile Asp Pro Phe Asp Asp
   980 985 990
   Arg Arg Glu Ala Gly Gly Glu Ser Thr Val Val Asn Pro Lys Ser Gly
   995 1000 1005
   Ser Trp Asn Phe Phe Asp Trp Phe Ser Gly Leu Met Ser Trp Phe
   1010 1015 1020
   Gly Gly Pro Leu Lys Thr Ile Leu Leu Ile Cys Leu Tyr Val Ala
   1025 1030 1035
   Leu Ser Ile Gly Leu Phe Phe Leu Leu Ile Tyr Leu Gly Arg Thr
   1040 1045 1050
   Gly Leu Ser Lys Met Trp Leu Ala Ala Thr Lys Lys Ala Ser
   1055 1060 1065
<210> 3
   <211> 853
   <212> PRT
   <213> HIV 89.6 Envelope Glycoprotein
<400> 3
   Met Arg Val Lys Glu Ile Arg Lys Asn Trp Gln His Leu Arg Gly Gly
   1 5 10 15
   Ile Leu Leu Leu Gly Met Leu Met Ile Cys Ser Ala Ala Lys Glu Lys
   20 25 30
   Thr Trp Val Thr Ile Tyr Tyr Gly Val Pro Val Trp Arg Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Gln Glu Val Val Leu Gly Asn Val Thr Glu Asn Phe Asn Met Trp Lys
                   85                  90                  95
   Asn Asn Met Val Asp Gln Met His Glu Asp Ile Ile Ser Leu Trp Asp
               100                 105                 110
   Glu Ser Leu Lys Pro Cys Val Lys Leu Thr Pro Leu Cys Val Thr Leu
           115                 120                 125
   Asn Cys Thr Asn Leu Asn Ile Thr Lys Asn Thr Thr Asn Pro Thr Ser
       130                 135                 140
   Ser Ser Trp Gly Met Met Glu Lys Gly Glu Ile Lys Asn Cys Ser Phe
   145                 150                 155                 160
   Tyr Ile Thr Thr Ser Ile Arg Asn Lys Val Lys Lys Glu Tyr Ala Leu
                   165                 170                 175
   Phe Asn Arg Leu Asp Val Val Pro Ile Glu Asn Thr Asn Asn Thr Lys
               180                 185                 190
   Tyr Arg Leu Ile Ser Cys Asn Thr Ser Val Ile Thr Gln Ala Cys Pro
           195                 200                 205
   Lys Val Ser Phe Gln Pro Ile Pro Ile His Tyr Cys Val Pro Ala Gly
       210                 215                 220
   Phe Ala Met Leu Lys Cys Asn Asn Lys Thr Phe Asn Gly Ser Gly Pro
   225 230 235 240
   Cys Thr Asn Val Ser Thr Val Gln Cys Thr His Gly Ile Arg Pro Val
   245 250 255
   Val Ser Thr Gln Leu Leu Leu Asn Gly Ser Leu Ala Glu Glu Asp Ile
   260 265 270
   Val Ile Arg Ser Glu Asn Phe Thr Asp Asn Ala Lys Thr Ile Ile Val
   275 280 285
   Gln Leu Asn Glu Ser Val Val Ile Asn Cys Thr Arg Pro Asn Asn Asn
   290 295 300
   Thr Arg Arg Arg Leu Ser Ile Gly Pro Gly Arg Ala Phe Tyr Ala Arg
   305 310 315 320
   Arg Asn Ile Ile Gly Asp Ile Arg Gln Ala His Cys Asn Ile Ser Arg
   325 330 335
   Ala Lys Trp Asn Asn Thr Leu Gln Gln Ile Val Ile Lys Leu Arg Glu
   340 345 350
   Lys Phe Arg Asn Lys Thr Ile Ala Phe Asn Gln Ser Ser Gly Gly Asp
   355 360 365
   Pro Glu Ile Val Met His Ser Phe Asn Cys Gly Gly Glu Phe Phe Tyr
   370 375 380
   Cys Asn Thr Ala Gln Leu Phe Asn Ser Thr Trp Asn Val Thr Gly Gly
   385 390 395 400
   Thr Asn Gly Thr Glu Gly Asn Asp Ile Ile Thr Leu Gln Cys Arg Ile
   405 410 415
   Lys Gln Ile Ile Asn Met Trp Gln Lys Val Gly Lys Ala Met Tyr Ala
               420                     425                     430
   Pro Pro Ile Thr Gly Gln Ile Arg Cys Ser Ser Asn Ile Thr Gly Leu
           435                     440                     445
   Leu Leu Thr Arg Asp Gly Gly Asn Ser Thr Glu Thr Glu Thr Glu Ile
       450                     455                     460
   Phe Arg Pro Gly Gly Gly Asp Met Arg Asp Asn Trp Arg Ser Glu Leu
   465                     470                     475                     480
   Tyr Lys Tyr Lys Val Val Arg Ile Glu Pro Ile Gly Val Ala Pro Thr
                   485                     490                     495
   Arg Ala Lys Arg Arg Thr Val Gln Arg Glu Lys Arg Ala Val Gly Ile
               500                     505                     510
   Gly Ala Val Phe Leu Gly Phe Leu Gly Ala Ala Gly Ser Thr Met Gly
           515                     520                     525
   Ala Ala Ser Val Thr Leu Thr Val Gln Ala Arg Leu Leu Leu Ser Gly
       530                     535                     540
   Ile Val Gln Gln Gln Asn Asn Leu Leu Arg Ala Ile Glu Ala Gln Gln
   545                     550                     555                     560
   His Met Leu Gln Leu Thr Val Trp Gly Ile Lys Gln Leu Gln Ala Arg
                   565                     570                     575
   Val Leu Ala Leu Glu Arg Tyr Leu Arg Asp Gln Gln Leu Met Gly Ile
               580                     585                     590
   Trp Gly Cys Ser Gly Lys Leu Ile Cys Thr Thr Ser Val Pro Trp Asn
           595                     600                     605
   Val Ser Trp Ser Asn Lys Ser Val Asp Asp Ile Trp Asn Asn Met Thr
       610                 615                 620
   Trp Met Glu Trp Glu Arg Glu Ile Asp Asn Tyr Thr Asp Tyr Ile Tyr
   625                 630                 635                 640
   Asp Leu Leu Glu Lys Ser Gln Thr Gln Gln Glu Lys Asn Glu Lys Glu
                   645                 650                 655
   Leu Leu Glu Leu Asp Lys Trp Ala Ser Leu Trp Asn Trp Phe Asp Ile
               660                 665                 670
   Thr Asn Trp Leu Trp Tyr Ile Arg Leu Phe Ile Met Ile Val Gly Gly
           675                 680                 685
   Leu Ile Gly Leu Arg Ile Val Phe Ala Val Leu Ser Ile Val Asn Arg
       690                 695                 700
   Val Arg Gln Gly Tyr Ser Pro Leu Ser Phe Gln Thr Leu Leu Pro Ala
   705                 710                 715                 720
   Ser Arg Gly Pro Asp Arg Pro Glu Gly Thr Glu Glu Glu Gly Gly Glu
                   725                 730                 735
   Arg Asp Arg Asp Arg Ser Gly Pro Leu Val Asn Gly Phe Leu Ala Leu
               740                 745                 750
   Phe Trp Val Asp Leu Arg Asn Leu Cys Leu Phe Leu Tyr His Leu Leu
           755                 760                 765
   Arg Asn Leu Leu Leu Ile Val Thr Arg Ile Val Glu Leu Leu Gly Arg
       770                 775                 780
   Arg Gly Trp Glu Ala Leu Lys Tyr Trp Trp Asn Leu Leu Gln Tyr Trp
   785                 790                 795                 800
   Ser Gln Glu Leu Lys Asn Ser Ala Val Ser Leu Leu Asn Ala Thr Ala
   805 810 815
   Ile Ala Val Ala Glu Gly Thr Asp Arg Val Ile Lys Ile Val Gln Arg
   820 825 830
   Ala Cys Arg Ala Ile Arg Asn Ile Pro Thr Arg Ile Arg Gln Gly Leu
   835 840 845
   Glu Arg Ala Leu Leu
   850
<210> 4
   <211> 507
   <212> PRT
   <213> RVFV GC
<400> 4
   Cys Ser Glu Leu Ile Gln Ala Ser Ser Arg Ile Thr Thr Cys Ser Thr
   1 5 10 15
   Glu Gly Val Asn Thr Lys Cys Arg Leu Ser Gly Thr Ala Leu Ile Arg
   20 25 30
   Ala Gly Ser Val Gly Ala Glu Ala Cys Leu Met Leu Lys Gly Val Lys
   35 40 45
   Glu Asp Gln Thr Lys Phe Leu Lys Ile Lys Thr Val Ser Ser Glu Leu
   50 55 60
   Ser Cys Arg Glu Gly Gln Ser Tyr Trp Thr Gly Ser Ile Ser Pro Lys
   65 70 75 80
   Cys Leu Ser Ser Arg Arg Cys His Leu Val Gly Glu Cys His Val Asn
   85 90 95
   Arg Cys Leu Ser Trp Arg Asp Asn Glu Thr Ser Ala Glu Phe Ser Phe
               100                 105                 110
   Val Gly Glu Ser Thr Thr Met Arg Glu Asn Lys Cys Phe Glu Gln Cys
           115                 120                 125
   Gly Gly Trp Gly Cys Gly Cys Phe Asn Val Asn Pro Ser Cys Leu Phe
       130                 135                 140
   Val His Thr Tyr Leu Gln Ser Val Arg Lys Glu Ala Leu Arg Val Phe
   145                 150                 155                 160
   Asn Cys Ile Asp Trp Val His Lys Leu Thr Leu Glu Ile Thr Asp Phe
                   165                 170                 175
   Asp Gly Ser Val Ser Thr Ile Asp Leu Gly Ala Ser Ser Ser Arg Phe
               180                 185                 190
   Thr Asn Trp Gly Ser Val Ser Leu Ser Leu Asp Ala Glu Gly Ile Ser
           195                 200                 205
   Gly Ser Asn Ser Phe Ser Phe Ile Glu Ser Pro Ser Lys Gly Tyr Ala
       210                 215                 220
   Ile Val Asp Glu Pro Phe Ser Glu Ile Pro Arg Gln Gly Phe Leu Gly
   225                 230                 235                 240
   Glu Ile Arg Cys Asn Ser Glu Ser Ser Val Leu Ser Ala His Glu Ser
                   245                 250                 255
   Cys Leu Arg Ala Pro Asn Leu Ile Ser Tyr Lys Pro Met Ile Asp Gln
               260                 265                 270
   Leu Glu Cys Thr Thr Asn Leu Ile Asp Pro Phe Val Val Phe Glu Arg
           275                 280                 285
   Gly Ser Leu Pro Gln Thr Arg Asn Asp Lys Thr Phe Ala Ala Ser Lys
       290                 295                 300
   Gly Asn Arg Gly Val Gln Ala Phe Ser Lys Gly Ser Val Gln Ala Asp
   305                 310                 315                 320
   Leu Thr Leu Met Phe Asp Asn Phe Glu Val Asp Phe Val Gly Ala Ala
                   325                 330                 335
   Val Ser Cys Asp Ala Ala Phe Leu Asn Leu Thr Gly Cys Tyr Ser Cys
               340                 345                 350
   Asn Ala Gly Ala Arg Val Cys Leu Ser Ile Thr Ser Thr Gly Thr Gly
           355                 360                 365
   Ser Leu Ser Ala His Asn Lys Asp Gly Ser Leu His Ile Val Leu Pro
       370                 375                 380
   Ser Glu Asn Gly Thr Lys Asp Gln Cys Gln Ile Leu His Phe Thr Val
   385                 390                 395                 400
   Pro Glu Val Glu Glu Glu Phe Met Tyr Ser Cys Asp Gly Asp Glu Arg
                   405                 410                 415
   Pro Leu Leu Val Lys Gly Thr Leu Ile Ala Ile Asp Pro Phe Asp Asp
               420                 425                 430
   Arg Arg Glu Ala Gly Gly Glu Ser Thr Val Val Asn Pro Lys Ser Gly
           435                 440                 445
   Ser Trp Asn Phe Phe Asp Trp Phe Ser Gly Leu Met Ser Trp Phe Gly
       450                 455                 460
   Gly Pro Leu Lys Thr Ile Leu Leu Ile Cys Leu Tyr Val Ala Leu Ser
   465                 470                 475                 480
   Ile Gly Leu Phe Phe Leu Leu Ile Tyr Leu Gly Arg Thr Gly Leu Ser
   485 490 495
   Lys Met Trp Leu Ala Ala Thr Lys Lys Ala Ser
   500 505
<210> 5
   <211> 527
   <212> PRT
   <213> RVFV GN
<400> 5
   Glu Asp Pro His Leu Arg Asn Arg Pro Gly Lys Gly His Asn Tyr Ile
   1 5 10 15
   Asp Gly Met Thr Gln Glu Asp Ala Thr Cys Lys Pro Val Thr Tyr Ala
   20 25 30
   Gly Ala Cys Ser Ser Phe Asp Val Leu Leu Glu Lys Gly Lys Phe Pro
   35 40 45
   Leu Phe Gln Ser Tyr Ala His His Arg Thr Leu Leu Glu Ala Val His
   50 55 60
   Asp Thr Ile Ile Ala Lys Ala Asp Pro Pro Ser Cys Asp Leu Gln Ser
   65 70 75 80
   Ala His Gly Asn Pro Cys Met Lys Glu Lys Leu Val Met Lys Thr His
   85 90 95
   Cys Pro Asn Asp Tyr Gln Ser Ala His Tyr Leu Asn Asn Asp Gly Lys
   100 105 110
   Met Ala Ser Val Lys Cys Pro Pro Lys Tyr Gly Leu Thr Glu Asp Cys
   115 120 125
   Asn Phe Cys Arg Gln Met Thr Gly Ala Ser Leu Lys Lys Gly Ser Tyr
   130 135 140
   Pro Leu Gln Asp Leu Phe Cys Gln Ser Ser Glu Asp Asp Gly Ser Lys
   145 150 155 160
   Leu Lys Thr Lys Met Lys Gly Val Cys Glu Val Gly Val Gln Ala His
   165 170 175
   Lys Lys Cys Asp Gly Gln Leu Ser Thr Ala His Glu Val Val Pro Phe
   180 185 190
   Ala Val Phe Lys Asn Ser Lys Lys Val Tyr Leu Asp Lys Leu Asp Leu
   195 200 205
   Lys Thr Glu Glu Asn Leu Leu Pro Asp Ser Phe Val Cys Phe Glu His
   210 215 220
   Lys Gly Gln Tyr Lys Gly Thr Met Asp Ser Gly Gln Thr Lys Arg Glu
   225 230 235 240
   Leu Lys Ser Phe Asp Ile Ser Gln Cys Pro Lys Ile Gly Gly His Gly
   245 250 255
   Ser Lys Lys Cys Thr Gly Asp Ala Ala Phe Cys Ser Ala Tyr Glu Cys
   260 265 270
   Thr Ala Gln Tyr Ala Asn Ala Tyr Cys Ser His Ala Asn Gly Ser Gly
   275 280 285
   Ile Val Gln Ile Gln Val Ser Gly Val Trp Lys Lys Pro Leu Cys Val
   290 295 300
   Gly Tyr Glu Arg Val Val Val Lys Arg Glu Leu Ser Ala Lys Pro Ile
   305 310 315 320
   Gln Arg Val Glu Pro Cys Thr Thr Cys Ile Thr Lys Cys Glu Pro His
                   325                 330                 335
   Gly Leu Val Val Arg Ser Thr Gly Phe Lys Ile Ser Ser Ala Val Ala
               340                 345                 350
   Cys Ala Ser Gly Val Cys Val Thr Gly Ser Gln Ser Pro Ser Thr Glu
           355                 360                 365
   Ile Thr Leu Lys Tyr Pro Gly Ile Ser Gln Ser Ser Gly Gly Asp Ile
       370                 375                 380
   Gly Val His Met Ala His Asp Asp Gln Ser Val Ser Ser Lys Ile Val
   385                 390                 395                 400
   Ala His Cys Pro Pro Gln Asp Pro Cys Leu Val His Gly Cys Ile Val
                   405                 410                 415
   Cys Ala His Gly Leu Ile Asn Tyr Gln Cys His Thr Ala Leu Ser Ala
               420                 425                 430
   Phe Val Val Val Phe Val Phe Ser Ser Ile Ala Ile Ile Cys Leu Ala
           435                 440                 445
   Val Leu Tyr Arg Val Leu Lys Cys Leu Lys Ile Ala Pro Arg Lys Val
       450                 455                 460
   Leu Asn Pro Leu Met Trp Ile Thr Ala Phe Ile Arg Trp Ile Tyr Lys
   465                 470                 475                 480
   Lys Met Val Ala Arg Val Ala His Asn Ile Asn Gln Val Asn Arg Glu
                   485                 490                 495
   Ile Gly Trp Met Glu Gly Gly Gln Leu Val Leu Gly Asn Pro Ala Pro
               500                 505                 510
   Ile Pro Arg His Ala Pro Ile Pro Arg Tyr Ser Thr Tyr Leu Met
           515                 520                 525
<210> 6
   <211> 26
   <212> PRT
   <213> N-terminus of the SIV Env Glyocoprotein-41 TM domain
<400> 6
   Tyr Ile Gln Tyr Gly Val Tyr Ile Val Val Gly Val Ile Leu Leu Arg
   1               5                   10                  15
   Ile Val Ile Tyr Ile Val Gln Met Leu Ala
               20                  25
<210> 7
   <211> 232
   <212> PRT
   <213> extracellular coding domain of the FL gene
<400> 7
   Met Thr Val Leu Ala Pro Ala Trp Ser Pro Asn Ser Ser Leu Leu Leu
   1               5                   10                  15
   Leu Leu Leu Leu Leu Ser Pro Cys Leu Arg Gly Thr Pro Asp Cys Tyr
               20                  25                  30
   Phe Ser His Ser Pro Ile Ser Ser Asn Phe Lys Val Lys Phe Arg Glu
           35                  40                  45
   Leu Thr Asp His Leu Leu Lys Asp Tyr Pro Val Thr Val Ala Val Asn
       50                  55                  60
   Leu Gln Asp Glu Lys His Cys Lys Ala Leu Trp Ser Leu Phe Leu Ala
   65                  70                  75                  80
   Gln Arg Trp Ile Glu Gln Leu Lys Thr Val Ala Gly Ser Lys Met Gln
                   85                  90                  95
   Thr Leu Leu Glu Asp Val Asn Thr Glu Ile His Phe Val Thr Ser Cys
   100 105 110
   Thr Phe Gln Pro Leu Pro Glu Cys Leu Arg Phe Val Gln Thr Asn Ile
   115 120 125
   Ser His Leu Leu Lys Asp Thr Cys Thr Gln Leu Leu Ala Leu Lys Pro
   130 135 140
   Cys Ile Gly Lys Ala Cys Gln Asn Phe Ser Arg Cys Leu Glu Val Gln
   145 150 155 160
   Cys Gln Pro Asp Ser Ser Thr Leu Leu Pro Pro Arg Ser Pro Ile Ala
   165 170 175
   Leu Glu Ala Thr Glu Leu Pro Glu Pro Arg Pro Arg Gln Leu Leu Leu
   180 185 190
   Leu Leu Leu Leu Leu Leu Pro Leu Thr Leu Val Leu Leu Ala Ala Ala
   195 200 205
   Trp Gly Leu Arg Trp Gln Arg Ala Arg Arg Arg Gly Glu Leu His Pro
   210 215 220
   Gly Val Pro Leu Pro Ser His Pro
   225 230
<210> 8
   <211> 164
   <212> PRT
   <213> SIV mac239 Env cytoplasmic domain
<400> 8
   Lys Leu Arg Gln Gly Tyr Arg Pro Val Phe Ser Ser Pro Pro Ser Tyr
   1 5 10 15
   Phe Gln Gln Thr His Ile Gln Gln Asp Pro Ala Leu Pro Thr Arg Glu
   20 25 30
   Gly Lys Glu Arg Asp Gly Gly Glu Gly Gly Gly Asn Ser Ser Trp Pro
   35 40 45
   Trp Gln Ile Glu Tyr Ile His Phe Leu Ile Arg Gln Leu Ile Arg Leu
   50 55 60
   Leu Thr Trp Leu Phe Ser Asn Cys Arg Thr Leu Leu Ser Arg Val Tyr
   65 70 75 80
   Gln Ile Leu Gln Pro Ile Leu Gln Arg Leu Ser Ala Thr Leu Gln Arg
   85 90 95
   Ile Arg Glu Val Leu Arg Thr Glu Leu Thr Tyr Leu Gln Tyr Gly Trp
   100 105 110
   Ser Tyr Phe His Glu Ala Val Gln Ala Val Trp Arg Ser Ala Thr Glu
   115 120 125
   Thr Leu Ala Gly Ala Trp Gly Asp Leu Trp Glu Thr Leu Arg Arg Gly
   130 135 140
   Gly Arg Trp Ile Leu Ala Ile Pro Arg Arg Ile Arg Gln Gly Leu Glu
   145 150 155 160
   Leu Thr Leu Leu
<210> 9
   <211> 23
   <212> PRT
   <213> tPA signal peptide
<400> 9
   Met Asp Ala Met Lys Arg Gly Leu Cys Cys Val Leu Leu Leu Cys Gly
   1 5 10 15
   Ala Val Phe Val Ser Ala Arg
   20
<210> 10
   <211> 40
   <212> PRT
   <213> GPI-anchoring seq. access no. x52645
<400> 10
   Leu Ala Val Ser Thr Ile Ser Ser Phe Phe Pro Pro Gly Tyr Gln Val
   1 5 10 15
   Ser Phe Cys Leu Val Met Val Leu Leu Phe Ala Val Asp Thr Gly Leu
   20 25 30
   Tyr Phe Ser Val Lys Thr Asn Ile
   35 40
<210> 11
   <211> 300
   <212> PRT
   <213> C3d sequence
<400> 11
   Thr Val Ile Ala Val His Tyr Leu Asp Gln Thr Glu Gln Trp Glu Lys
   1 5 10 15
   Phe Gly Ile Glu Lys Arg Gln Glu Ala Leu Glu Leu Ile Lys Lys Gly
   20 25 30
   Tyr Thr Gln Gln Leu Ala Phe Lys Gln Pro Ser Ser Ala Tyr Ala Ala
   35 40 45
   Phe Asn Asn Arg Pro Pro Ser Thr Trp Leu Thr Ala Tyr Val Val Lys
   50 55 60
   Val Phe Ser Leu Ala Ala Asn Leu Ile Ala Ile Asp Ser His Val Leu
   65                  70                  75                  80
   Cys Gly Ala Val Lys Trp Leu Ile Leu Glu Lys Gln Lys Pro Asp Gly
                   85                  90                  95
   Val Phe Gln Glu Asp Gly Pro Val Ile His Gln Glu Met Ile Gly Gly
               100                 105                 110
   Phe Arg Asn Ala Lys Glu Ala Asp Val Ser Leu Thr Ala Phe Val Leu
           115                 120                 125
   Ile Ala Leu Gln Glu Ala Arg Asp Ile Cys Glu Gly Gln Val Asn Ser
       130                 135                 140
   Leu Pro Gly Ser Ile Asn Lys Ala Gly Glu Tyr Ile Glu Ala Ser Tyr
   145                 150                 155                 160
   Met Asn Leu Gln Arg Pro Tyr Thr Val Ala Ile Ala Gly Tyr Ala Leu
                   165                 170                 175
   Ala Leu Met Asn Lys Leu Glu Glu Pro Tyr Leu Gly Lys Phe Leu Asn
               180                 185                 190
   Thr Ala Lys Asp Arg Asn Arg Trp Glu Glu Pro Asp Gln Gln Leu Tyr
           195                 200                 205
   Asn Val Glu Ala Thr Ser Tyr Ala Leu Leu Ala Leu Leu Leu Leu Lys
       210                 215                 220
   Asp Phe Asp Ser Val Pro Pro Val Val Arg Trp Leu Asn Glu Gln Arg
   225                 230                 235                 240
   Tyr Tyr Gly Gly Gly Tyr Gly Ser Thr Gln Ala Thr Phe Met Val Phe
                   245                 250                 255
   Gln Ala Leu Ala Gln Tyr Gln Thr Asp Val Pro Asp His Lys Asp Leu
               260                 265                 270
   Asn Met Asp Val Ser Phe His Leu Pro Ser Arg Ser Ser Ala Thr Thr
           275                 280                 285
   Phe Arg Leu Leu Trp Glu Asn Gly Asn Leu Leu Arg
       290                 295                 300